# EUROPEAN PATENT APPLICATION

(11) **EP 4 811 038 A1**
(43) Date of publication of application: **23.09.2026**
(21) Application number: 24897489.1
(22) Date of filing: 25.11.2024
(51) Int. Cl.: G03F 7/004, C07C 309/80, C07C 381/12, C08F 220/12, C09K 3/00, G03F 7/039

(54) **RADIATION-SENSITIVE RESIN COMPOSITION, PATTERN FORMATION METHOD, METHOD FOR PRODUCING ELECTRONIC DEVICE, ACID GENERATION AGENT, METHOD FOR PRODUCING COMPOUND, COMPOUND, AND ACID GENERATION METHOD**

(30) Priority: 30.11.2023 JP 2023202572
(71) Applicant: Central Glass Company, Limited, Ube-shi, Yamaguchi 755-0001 (JP)
(72) Inventor: MASUBUCHI, Takashi, Tokyo 101-0054 (JP); IMAIZUMI, Yuki, Tokyo 101-0054 (JP); MIURA, Masahiro, Tokyo 101-0054 (JP); FUJIMOTO, Masataka, Tokyo 101-0054 (JP); MASUDA, Takashi, Tokyo 101-0054 (JP); HONDA, Yuki, Tokyo 101-0054 (JP); KANEKO, Yuzuru, Tokyo 101-0054 (JP)
(74) Representative: Manitz Finsterwald Patent- und Rechtsanwaltspartnerschaft mbB
(86) International application number: PCT/JP2024/041628
(87) International publication number: WO 2025/115804

(57) **Abstract**

A radiation-sensitive resin composition including an acid generator represented by General Formula (AG1). In General Formula (AG1), R¹ and R² each independently represent a fluorine atom or -OR, where R is a monovalent organic group, and A⁺ is a counter cation. In addition, an acid generator represented by General Formula (AG1). In addition, a compound represented by General Formula (AG1).

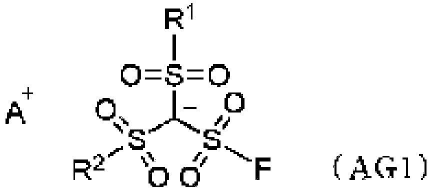

## Description

### TECHNICAL FIELD

The present invention relates to a radiation-sensitive resin composition, a pattern formation method, a method for manufacturing an electronic device, an acid generator, a method for producing a compound, a compound, and a method for generating an acid.

### BACKGROUND ART

In the manufacturing of an electronic device, a radiation-sensitive resin composition such as a photoresist is used in order to form a fine pattern on a substrate. Typically, a resin film formed on a substrate using a radiation-sensitive resin composition is irradiated with radiation and subjected to a development treatment to form a pattern on the substrate. A fine circuit is formed on the substrate by performing dry etching using the pattern as a mask.

With the progress of the miniaturization and complexity of electronic devices, the improvement of the radiation-sensitive resin composition has also been continued.

Many radiation-sensitive resin compositions for forming a fine pattern on a nanometer order are chemically amplified. In a chemically amplified radiation-sensitive resin composition, one acid generated from an acid generator is involved in the solubilization or insolubilization reaction of the composition in a catalytic manner a plurality of times. Therefore, a chemically amplified radiation-sensitive resin composition is easily designed to have a relatively high sensitivity.

There are two types of chemically amplified radiation-sensitive resin compositions, which are classified as follows.
(i) a composition containing a resin that is decomposed by the action of an acid to change the solubility in a developer, and a compound (acid generator) that generates an acid by irradiation with radiation
(ii) a composition containing a component (for example, a resin and a crosslinking agent) that increases in molecular weight by the action of an acid to change the solubility in a developer, and an acid generator

Patent Document 1 describes, for example, an energy-active salt having an anion represented by (CF₃SO₂)₃C⁻. This energy-active salt can be used as an acid generator in a radiation-sensitive resin composition.

Patent Document 2 describes a radiation-sensitive resin composition containing an acid generator having a cation structure.

Patent Document 3 also describes various acid generators that can be applied to a radiation-sensitive resin composition.

Many acid generators contain a fluorine atom having a high electronegativity. This is to increase the acid strength of the generated acid and to increase the sensitivity of the radiation-sensitive resin composition.

### RELATED DOCUMENT

### PATENT DOCUMENT

Patent Document 1: Japanese Patent No. 3985020
Patent Document 2: Japanese Patent No. 5645510
Patent Document 3: Japanese Unexamined Patent Publication No. 2002-341539

### SUMMARY OF THE INVENTION

### TECHNICAL PROBLEM

As described above, with the progress of the miniaturization and complexity of electronic devices, the improvement of the radiation-sensitive resin composition has been continued. With the improvement study of the radiation-sensitive resin composition, there is a potential need for a novel acid generator that can improve some performance of the radiation-sensitive resin composition.

The present inventors have conducted studies with one of the objects of providing a novel acid generator and a radiation-sensitive resin composition containing the same.

### SOLUTION TO PROBLEM

The present inventors have completed the following radiation-sensitive resin composition and the like.
1. A radiation-sensitive resin composition including an acid generator represented by General Formula (AG1), 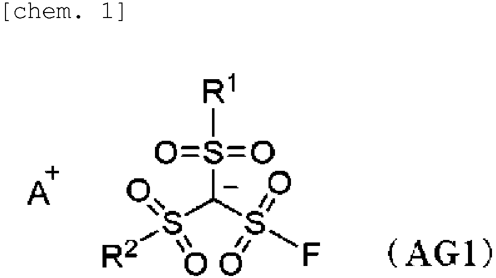 in General Formula (AG1),
   R¹ and R² each independently represent a fluorine atom or - OR, where R is a monovalent organic group, and
   A⁺ is a counter cation.
2. The radiation-sensitive resin composition according to 1., further including
   a resin which is decomposed by an action of an acid to change solubility in a developer.
3. The radiation-sensitive resin composition according to 1., further including a component that increases in molecular weight due to an action of an acid and changes solubility in a developer.
4. The radiation-sensitive resin composition according to any one of 1. to 3.,
   in which R¹ and R² are both fluorine atoms.
5. The radiation-sensitive resin composition according to any one of 1. to 4,
   in which one of R¹ or R² is a fluorine atom and the other is -OR.
6. The radiation-sensitive resin composition according to any one of 1. to 5.,
   in which at least one of R¹ or R² is -OR, and R includes a cyclic skeleton.
7. The radiation-sensitive resin composition according to any one of 1. to 6.,
   in which A⁺ is at least any cation selected from the group consisting of a sulfonium cation and an iodonium cation.
8. A pattern formation method including:
   forming a film using the radiation-sensitive resin composition according to any one of 1. to 7.;
   irradiating the film with radiation; and
   developing the film irradiated with the radiation.
9. A method for manufacturing an electronic device, the method including the pattern formation method according to 8..
10. An acid generator represented by General Formula (AG1), in which 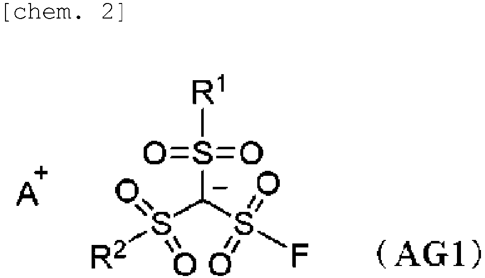 in General Formula (AG1),
   R¹ and R² each independently represent a fluorine atom or - OR, where R is a monovalent organic group, and
   A⁺ is a counter cation.
11. The acid generator according to 10.,
   in which both R¹ and R² represent a fluorine atom.
12. The acid generator according to 10. or 11.,
   in which one of R¹ or R² represents a fluorine atom and the other represents -OR.
13. The acid generator according to 10.,
   in which at least one of R¹ or R² represents -OR, and R includes a cyclic skeleton.
14. The acid generator according to any one of 10. to 13.,
   in which A⁺ represents at least any cation selected from the group consisting of a sulfonium cation and an iodonium cation.
15. A method for producing a compound, the method including reacting a compound represented by General Formula (ag1) with a compound represented by General Formula A₁⁺X⁻ (A₁⁺ is a sulfonium cation or an iodonium cation, and X⁻ is a halide ion, a sulfonate ion, a sulfate ion, a phosphate ion, a hydroxide ion, or a carboxylate ion) to produce a compound represented by General Formula (AG1-1), in which 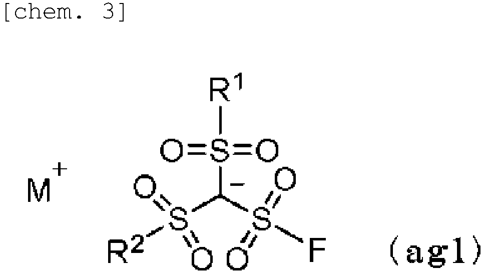 in General Formula (ag1),
   M⁺ is a metal cation,
   R¹ and R² each independently represent a fluorine atom or - OR, where R is a monovalent organic group, 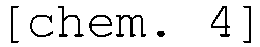 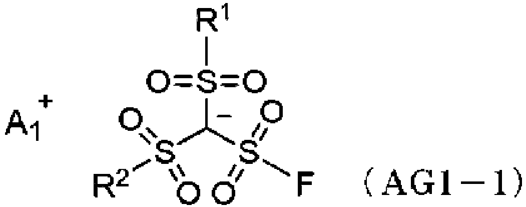
   in General Formula (AG1-1),
   a definition of A₁⁺ is as described above,
   R¹ and R² each independently represent a fluorine atom or - OR, where R is a monovalent organic group.
16. A method for producing a compound, the method including reacting a compound represented by General Formula (M-FSM) with a compound represented by R-OH, where R is a monovalent organic group, to obtain a compound represented by General Formula (ag1), in which 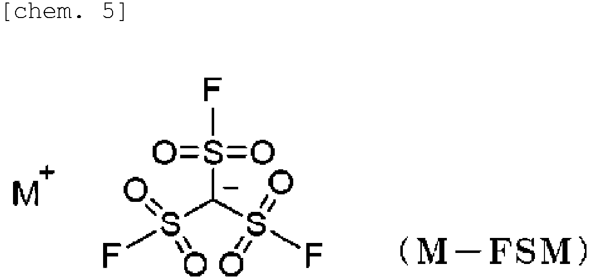 in General Formula (M-FSM),
   M⁺ is a metal cation, 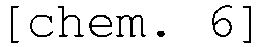 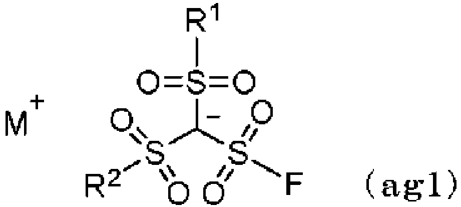
   in General Formula (ag1),
   M⁺ is a metal cation,
   R¹ and R² each independently represent a fluorine atom or - OR, where at least one of R¹ or R² is -OR, and R is a monovalent organic group.
17. A compound represented by General Formula (AG1), in which 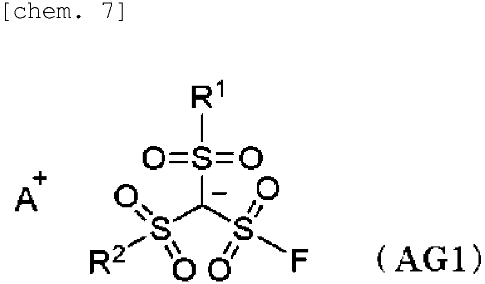 in General Formula (AG1),
   R¹ and R² each independently represent a fluorine atom or - OR, where R is a monovalent organic group, and
   A⁺ is a counter cation.
18. The compound according to 17.,
   in which both R¹ and R² represent a fluorine atom.
19. The compound according to 17. or 18.,
   in which one of R¹ or R² represents a fluorine atom, and the other represents -OR.
20. The compound according to 17.,
   in which at least one of R¹ or R² represents -OR, and R includes a cyclic skeleton.
21. The compound according to any one of 17. to 20.,
   in which A⁺ represents at least any cation selected from the group consisting of a sulfonium cation and an iodonium cation.
22. A method for generating an acid, the method including irradiating the compound according to any one of 17. to 21. with radiation to generate an acid.

### ADVANTAGEOUS EFFECTS OF INVENTION

The novel acid generators and radiation-sensitive resin compositions described above are useful in pattern formation in which a resin film is formed on a substrate and irradiated with radiation and subjected to a development treatment.

### DESCRIPTION OF EMBODIMENTS

Hereinafter, embodiments of the present invention will be described in detail.

In the present specification, the notation "X to Y" in the explanation of the range of values means equal to or more than X and equal to or less than Y, unless otherwise specified. For example, "1% to 5% by mass" means "1% by mass or more and 5% by mass or less".

In the present specification, the "non-volatile component" means a component other than a solvent in the radiation-sensitive resin composition unless otherwise specified.

In the present specification, the "radiation" means a ray or a particle beam capable of activating an acid generator. Typically, the radiation can be ultraviolet rays (particularly, far ultraviolet rays and extreme ultraviolet rays), X-rays, γ-rays, electron beams, or the like. In addition, in the present specification, the "light" is synonymous with the "radiation" unless otherwise specified.

In the present specification, the "acid generator" typically means a compound having a property of generating an acid by an external stimulus (energy from the outside) such as light or heat. For example, a compound represented by a certain general formula X but not generating an acid by an external stimulus does not correspond to the "acid generator represented by the general formula X". On the other hand, for example, the "compound represented by the general formula X" includes all compounds represented by the general formula X regardless of characteristics such as responsiveness to an external stimulus.

In a case where substitution or non-substitution is not explicitly indicated in the description of a group (atomic group) in the present specification, the group (atomic group) is intended to include both a group (atomic group) having no substituent and a group (atomic group) having a substituent. For example, the concept of an "alkyl group" includes not only an alkyl group not having a substituent (unsubstituted alkyl group) but also an alkyl group having a substituent (substituted alkyl group).

The expression "(meth)acryl" in the present specification represents a concept including both acryl and methacryl. The same applies to similar expressions such as "(meth)acrylate".

Unless otherwise specified, the term "organic group" as used in the present specification means an atomic group obtained by removing one or more hydrogen atoms from an organic compound. For example, a "monovalent organic group" refers to an atomic group obtained by removing one hydrogen atom from any organic compound.

The term "electronic device" in the present specification is used as a meaning including an element, a device, a final product, and the like, to which electronic engineering technology has been applied, such as a semiconductor chip, a semiconductor element, a printed circuit board, an electric circuit display device, an information communication terminal, a light emitting diode, a physical battery, and a chemical battery.

### <Radiation-sensitive resin composition>

The radiation-sensitive resin composition containing an acid generator represented by General Formula (AG1) is novel, and can be suitably applied to pattern formation in which a resin film is formed on a substrate and irradiated with radiation and subjected to a development treatment.

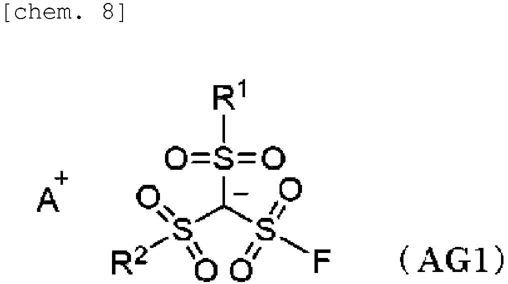

In General Formula (AG1),
R¹ and R² each independently represent a fluorine atom or - OR, where R is a monovalent organic group, and
A⁺ is a counter cation.

The advantages of configuring a radiation-sensitive resin composition using an acid generator represented by General Formula (AG1) will be described below in consideration of the findings in the related art.

The generated acid from the acid generator represented by General Formula (AG1) has a strong electron-withdrawing -SO₂F structure. Due to this, it is considered that the acid strength of the generated acid is increased. It is considered that this leads to an increase in the sensitivity of the radiation-sensitive resin composition.

In addition, according to the studies by the present inventors, the acid generator represented by General Formula (AG1) is stably present in organic solvent, but the generated acid from the acid generator represented by General Formula (AG1) is easily decomposed by being brought into contact with an alkaline developer (alkaline aqueous solution) (it is considered that the S-F bond is hydrolyzed). Therefore, it is considered that the unintended residue of the fluorine-containing component derived from the acid generator on the substrate is reduced in a case where a pattern is formed on the substrate using the acid generator represented by General Formula (AG1).

Incidentally, it is considered that the fact that the number of fluorine atoms in the anion moiety in General Formula (AG1) is originally relatively small also contributes to the reduction in the residue of the fluorine-containing component.

Many of the acid generators in the related art contain a fluorine atom, which is electron-withdrawing, in order to satisfy high resolution and low roughness and to increase the acid strength of the generated acid, thereby achieving higher sensitivity, in a case of being used in a radiation-sensitive resin composition. However, there is a concern that the unintended residue of the fluorine-containing component derived from the acid generator on the substrate may adversely affect the yield and performance of the electronic device. Specifically, there is a concern that the unintended residue of the fluorine-containing component may affect the rate of dry etching. That is, in a case where the fluorine-containing component derived from the radiation-sensitive resin composition is unintentionally residual on the substrate, there is a concern that appropriate dry etching cannot be performed. In addition, since many fluorine-containing compounds are water-repellent, there is also a concern that the unintended residue of the fluorine-containing compound on the substrate may unintentionally change the characteristics of the substrate surface. In particular, with the recent progress in the miniaturization and complexity of electronic devices, extremely small amounts of residues that have not been a problem so far may become a problem.

In order to reduce the unintended residue of the fluorine-containing compound on the substrate, it is considered to use an acid generator that does not contain a fluorine atom. However, many acid generators that do not contain a fluorine atom have a low acid strength, which may lead to a decrease in the sensitivity of the radiation-sensitive resin composition. That is, it can be understood that increasing the acid strength of the generated acid to increase the sensitivity of the radiation-sensitive resin composition and reducing the unintended residue of the fluorine-containing compound on the substrate are in a trade-off relationship.

Furthermore, in recent years, from the viewpoint of preventing environmental pollution and health damage, there is a case where it is required to reduce the amount of fluorine used.

It is considered that the above concerns and issues can be addressed by preparing a radiation-sensitive resin composition using an acid generator represented by General Formula (AG1). In particular, it is important to overcome the trade-off between increasing the acid strength of the generated acid to increase the sensitivity of the radiation-sensitive resin composition and reducing the unintended residue of the fluorine-containing compound on the substrate.

General Formula (AG1) will be described in more detail below.

### · Structure on anion side

As described above, in General Formula (AG1), R¹ and R² are each independently a fluorine atom or -OR, and R is a monovalent organic group.

It is preferable to appropriately select R¹ and R² from the viewpoint of the acid strength of the generated acid and the diffusibility of the generated acid.

From the viewpoint of increasing the acid strength of the generated acid, it is preferable that at least one of R¹ or R² is a fluorine atom, and it is more preferable that both R¹ and R² are fluorine atoms. As the acid strength of the generated acid increases, the sensitivity of the radiation-sensitive resin composition tends to improve.

From the viewpoint of controlling the diffusibility of the generated acid, it is preferable that at least one of R¹ or R² is -OR. By adjusting the molecular weight and bulkiness of R, the diffusibility of the generated acid can be variously adjusted. By appropriately adjusting the diffusibility of the generated acid, the performance of the radiation-sensitive resin composition, such as resolution and line edge roughness, tends to be improved.

R is not particularly limited as long as it is a monovalent organic group, but from the viewpoint of suppressing the diffusibility of the generated acid to improve the resolution and line edge roughness, it is preferable that R includes a cyclic skeleton.

More specifically, R can include a monocyclic or polycyclic aliphatic hydrocarbon group, a monocyclic or polycyclic aromatic hydrocarbon group, and the like. R may include a heterocyclic group, and the heterocyclic group may be aromatic or nonaromatic. Examples of the heteroatom included in the heterocyclic group include an oxygen atom, a nitrogen atom, and a sulfur atom.

R may be a group having only a cyclic skeleton as a chemical structure (for example, a monocyclic or polycyclic aliphatic hydrocarbon group or a monocyclic or polycyclic aromatic hydrocarbon group itself), or may be a group having a cyclic skeleton and a chemical structure other than the cyclic skeleton. Specifically, in the latter case, -OR may be -O-L-R'. Here, L is a divalent linking group, and R' is a monocyclic or polycyclic aliphatic hydrocarbon group, a monocyclic or polycyclic aromatic hydrocarbon group, or a heterocyclic group. Examples of the divalent linking group of L include a linear or branched alkylene group and a carbonyl group.

Examples of the monocyclic aliphatic hydrocarbon group include a cycloalkyl group and a cycloalkenyl group. Specific examples thereof include a cyclopropyl group, a cyclobutyl group, a cyclopentyl group, a cyclohexyl group, a cycloheptyl group, a cyclooctyl group, a cyclododecyl group, a cyclopentenyl group, a cyclohexenyl group, and a cyclooctadienyl group. Among these, a cyclopropyl group, a cyclopentyl group, a cyclohexyl group, or a cyclooctyl group is preferable.

Examples of the polycyclic aliphatic hydrocarbon group include a bicyclo[4.3.0]nonyl group, a decahydronaphthalenyl group, a tricyclo[5.2.1.0^{2,6}]decanyl group, a bornyl group, an isobornyl group, a norbornyl group, an adamantyl group, a noradamantyl group, a 1,7,7-trimethyltricyclo[2.2.1.0^{2,6}]heptanyl group, a 3,7,7-trimethylbicyclo[4.1.0]heptanyl group, and a group having a steroid skeleton. Among these, a norbornyl group, an adamantyl group, or a noradamantyl group is preferable.

Examples of the aromatic hydrocarbon group include a phenyl group and a naphthyl group.

Examples of the heterocyclic group include a group having a lactone skeleton.

The monovalent organic group of R may or may not have a substituent. The substituent is not particularly limited. Examples of the substituent include an alkyl group, an alicyclic group, an aryl group, an aralkyl group, a hydroxy group, a carbonyl group, a cyano group, a halogeno group, an alkoxycarbonyl group, and an alkylcarbonyloxy group.

From the viewpoint of appropriate diffusibility of the generated acid, the number of carbon atoms in the monovalent organic group (preferably having a cyclic skeleton) of R is preferably 5 to 30 and more preferably 6 to 25.

From the viewpoint of balance between the acid strength of the generated acid and the diffusibility of the generated acid, it is preferable that one of R¹ and R² is a fluorine atom and the other is -OR.

Preferred structures of the anion side in the acid generator represented by General Formula (AG1) are shown below.

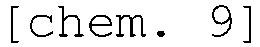

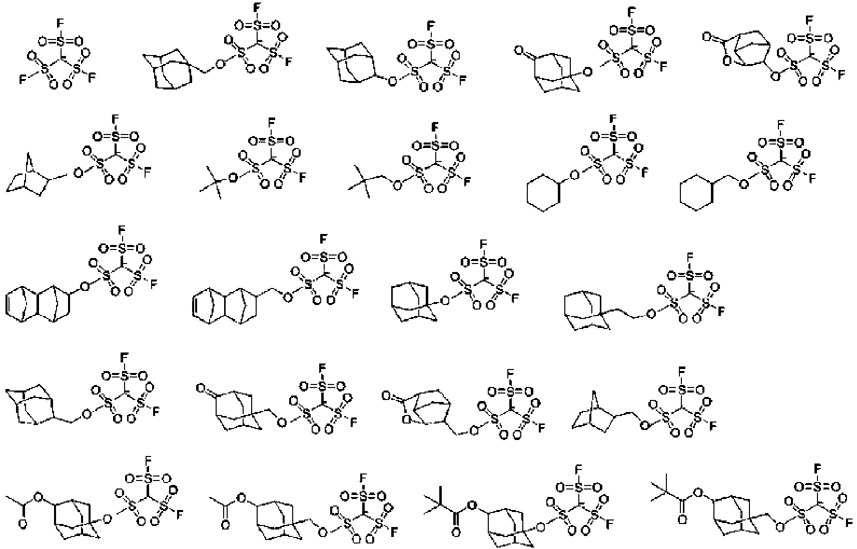

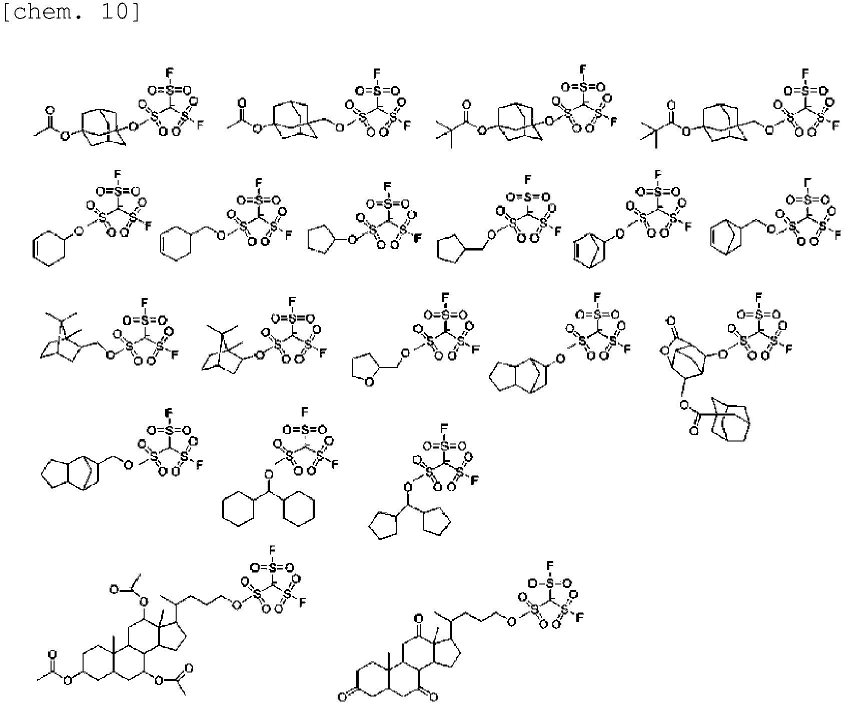

### · Structure of cation side

The structure of the counter cation of A⁺ is not particularly limited as long as the acid generator represented by General Formula (AG1) functions as an acid generator. By adopting A⁺ having an appropriate structure, for example, the sensitivity of the radiation-sensitive resin composition can be increased.

A⁺ is preferably an onium cation and more preferably at least any cation selected from the group consisting of a sulfonium cation and an iodonium cation.

More specifically, A⁺ is preferably represented by General Formula (A1) or (A2).

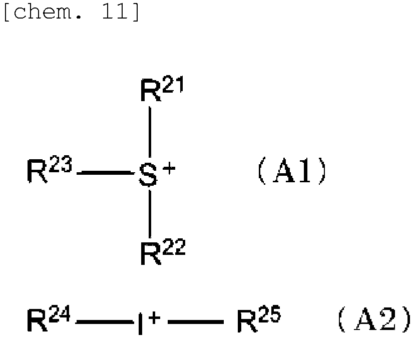

In General Formula (A1), R²¹, R²², and R²³ each independently represent an organic group. The number of carbon atoms in the organic group is usually 1 to 30 and preferably 1 to 20. At least two of R²¹, R²², and R²³ may be bonded to each other to form a ring structure. In addition, one or two or more selected from the group consisting of an oxygen atom, a sulfur atom, an ester bond, an amide bond, and a carbonyl group may be included in the ring structure. Preferred examples of the group formed by bonding at least two of R²¹, R²², and R²³ to each other include an alkylene group (for example, a butylene group and a pentylene group).

In General Formula (A2), R²⁴ and R²⁵ each independently represent an organic group. The number of carbon atoms in the organic group is usually 1 to 30 and preferably 1 to 20.

The organic groups of R²¹, R²², and R²³, and R²⁴ and R²⁵ can be an aryl group, an alkyl group, a cycloalkyl group, or the like.

The aryl group is preferably a phenyl group or a naphthyl group, and more preferably a phenyl group. The aryl group may be an aryl group having a heterocyclic structure having an oxygen atom, a nitrogen atom, a sulfur atom, or the like. Examples of the aryl group having a heterocyclic structure include a pyrrole residue, a furan residue, a thiophene residue, an indole residue, a benzofuran residue, and a benzothiophene residue. In General Formula (A1), in a case where two or more of R²¹, R²², and R²³ are aryl groups, the two or more aryl groups may be the same or different from each other. Similarly, in General Formula (A2), in a case where both R²⁴ and R²⁵ are aryl groups, the two aryl groups may be the same or different from each other.

Examples of the alkyl group or the cycloalkyl group include a linear or branched alkyl group having 1 to 15 carbon atoms and a cycloalkyl group having 3 to 15 carbon atoms. More specific examples thereof include a methyl group, an ethyl group, a propyl group, an n-butyl group, a sec-butyl group, a t-butyl group, a cyclopropyl group, a cyclobutyl group, and a cyclohexyl group.

The organic group constituting R²¹, R²², and R²³, and R²⁴ and R²⁵ may or may not have a substituent. Examples of the substituent include an alkyl group, a cycloalkyl group, an aryl group, an alkoxy group, an alkoxycarbonyl group, an alkylcarbonyloxy group, a halogeno group, a hydroxy group, and a phenylthio group. The preferred substituent is a linear or branched alkyl group having 1 to 12 carbon atoms, a cycloalkyl group having 3 to 12 carbon atoms, or a linear, branched, or cyclic alkoxy group having 1 to 12 carbon atoms, and more preferably an alkyl group having 1 to 4 carbon atoms or an alkoxy group having 1 to 4 carbon atoms.

In the cation structure represented by General Formula (A1), the substituent may be substituted on only one of R²¹, R²², and R²³, may be substituted on two of R²¹, R²², and R²³, or may be substituted on all three of R²¹, R²², and R²³. R²¹, R²², and R²³ may or may not have a substituent.

In the cation structure represented by General Formula (A2), the substituent may be substituted on only one of R²⁴ and R²⁵, or may be substituted on both R²⁴ and R²⁵. R²⁴ and R²⁵ may or may not have a substituent.

The total number of carbon atoms in R²¹ is not particularly limited, but from the viewpoint of the balance of various performances, it is, for example, 3 to 20, preferably 5 to 18, more preferably 6 to 16, and still more preferably 6 to 12. The same applies to the total number of carbon atoms in R²² and R²³, and R²⁴ and R²⁵.

From the viewpoint of stability in the radiation-sensitive resin composition, absorbability of radiation, and the like, in the cation structure represented by General Formula (A1), it is preferable that at least one of R²¹, R²², or R²³ is an aryl group, it is more preferable that at least two of R²¹, R²², and R²³ are aryl groups, and it is still more preferable that all of R²¹, R²², and R²³ are aryl groups.

Similarly, in the cation structure represented by General Formula (A2), it is preferable that at least one of R²⁴ or R²⁵ is an aryl group, and it is more preferable that both R²⁴ and R²⁵ are aryl groups.

Preferred structures of A⁺ in General Formula (AG1) are shown below. In the following, "Ph" represents a phenyl group.

In addition to the structures shown below, for example, cation structures of acid generators exemplified in paragraphs 0221 to 0227 and paragraphs 0241 to 0249 of JP2002-341539A can also be adopted as A⁺.

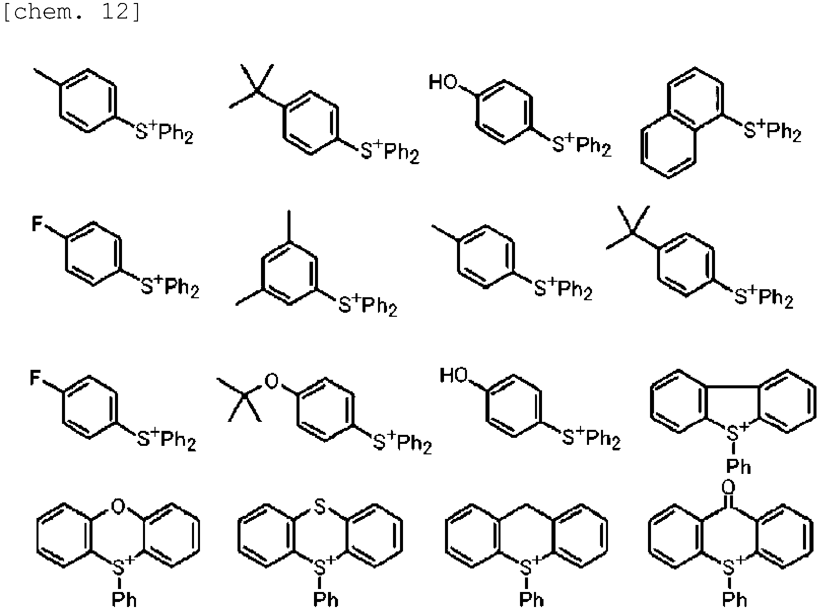

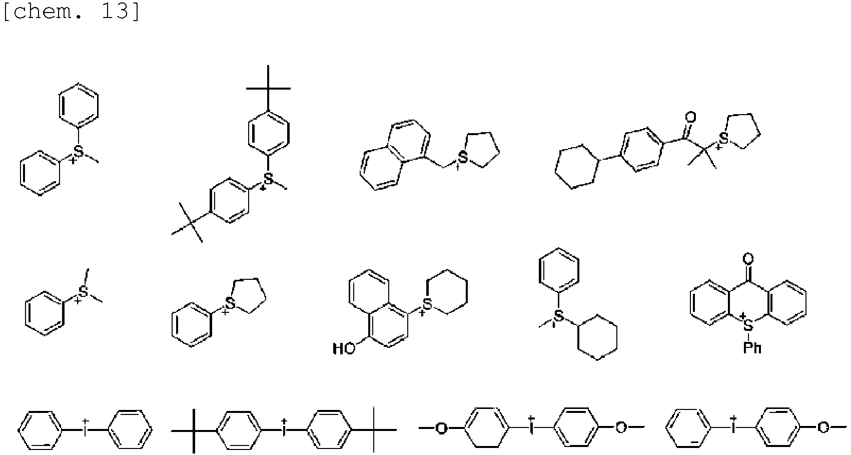

Specific preferred examples of the compound represented by General Formula (AG1) are shown below. In the following, "Me" represents a methyl group.

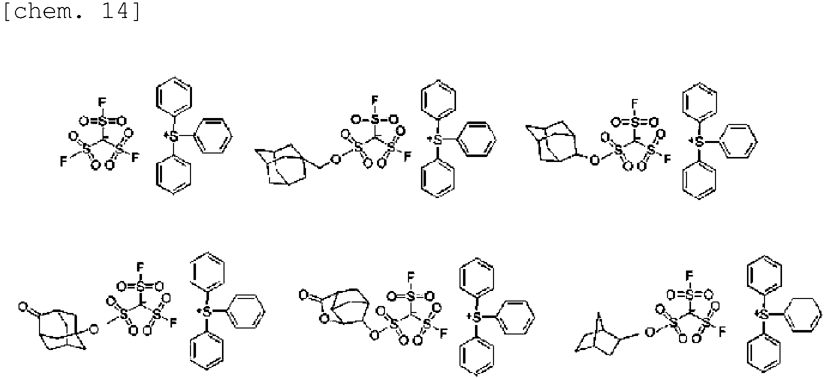

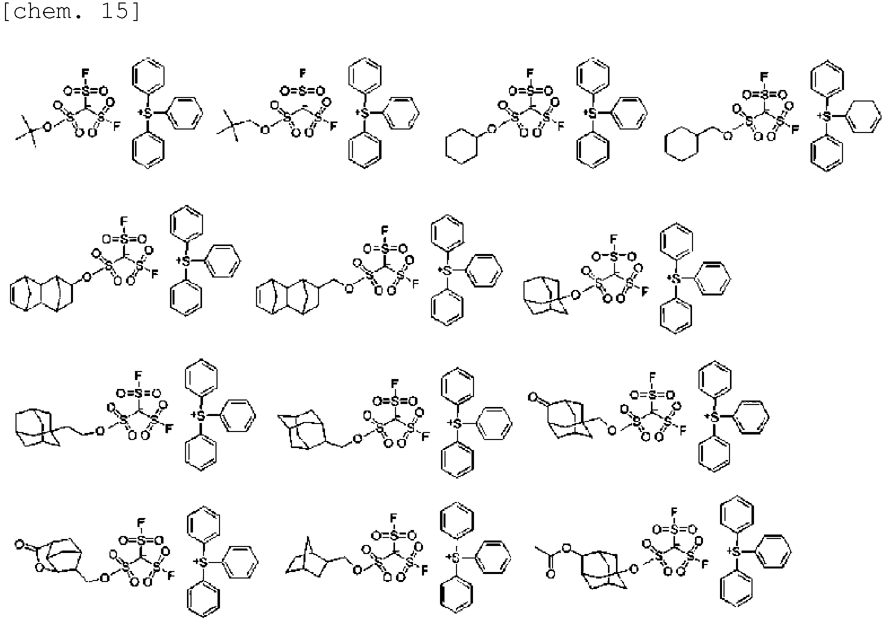

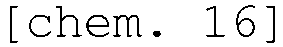

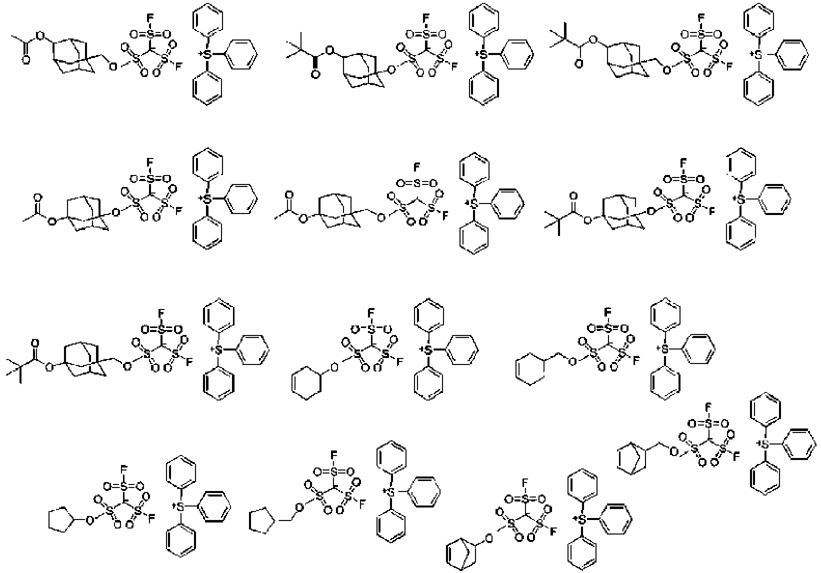

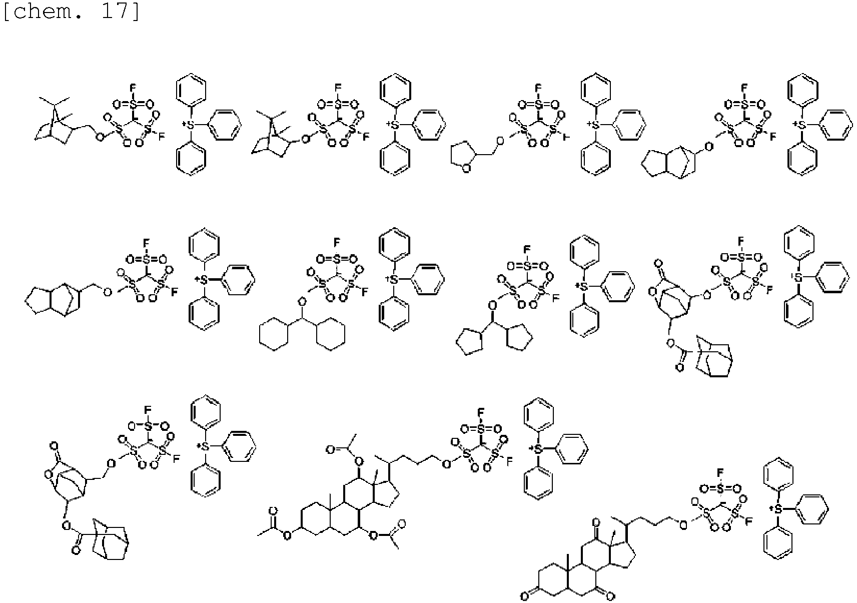

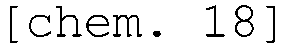

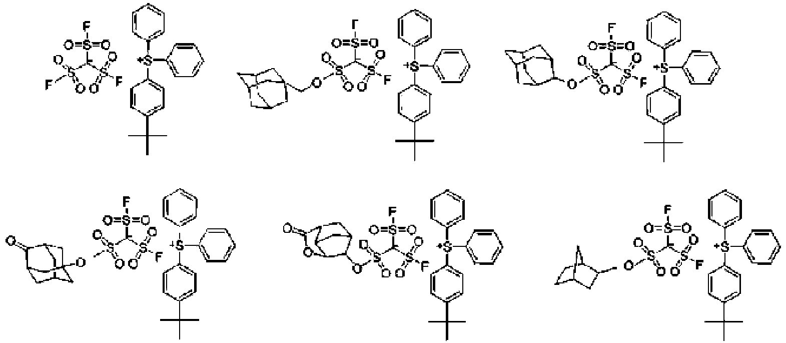

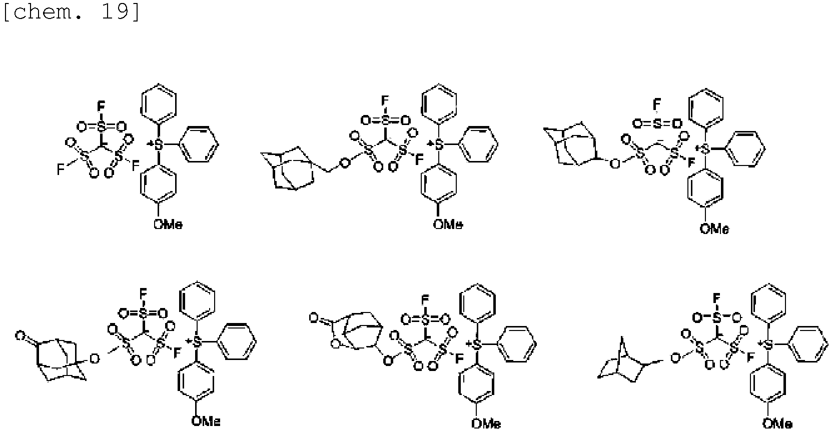

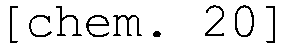

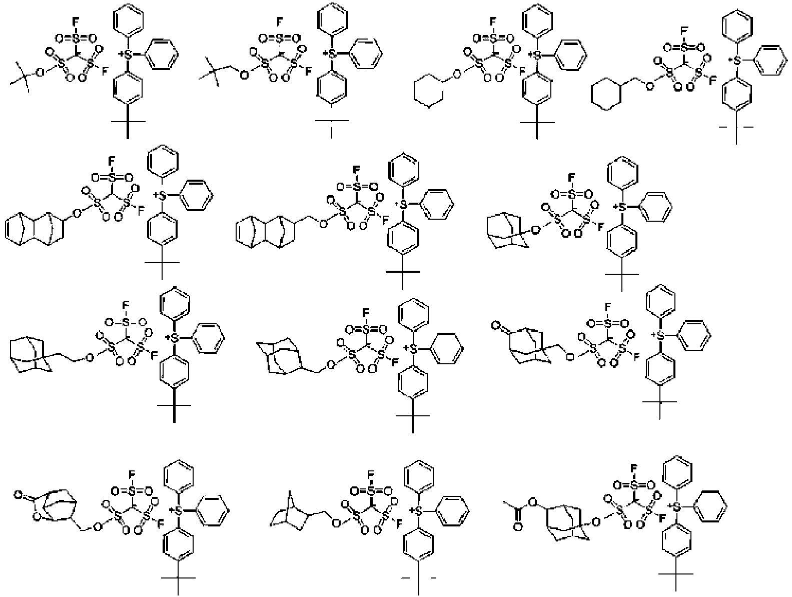

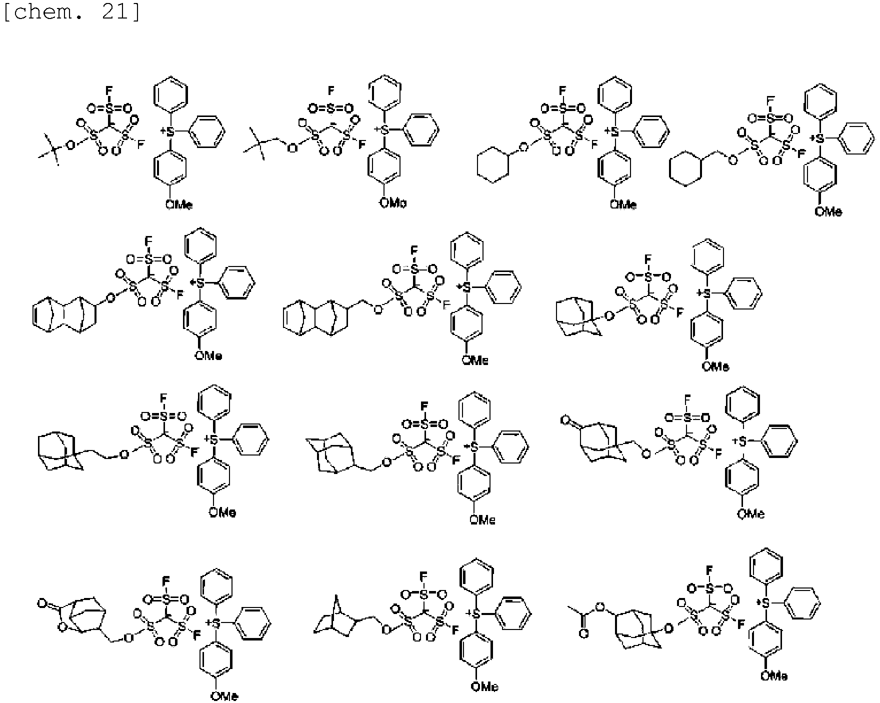

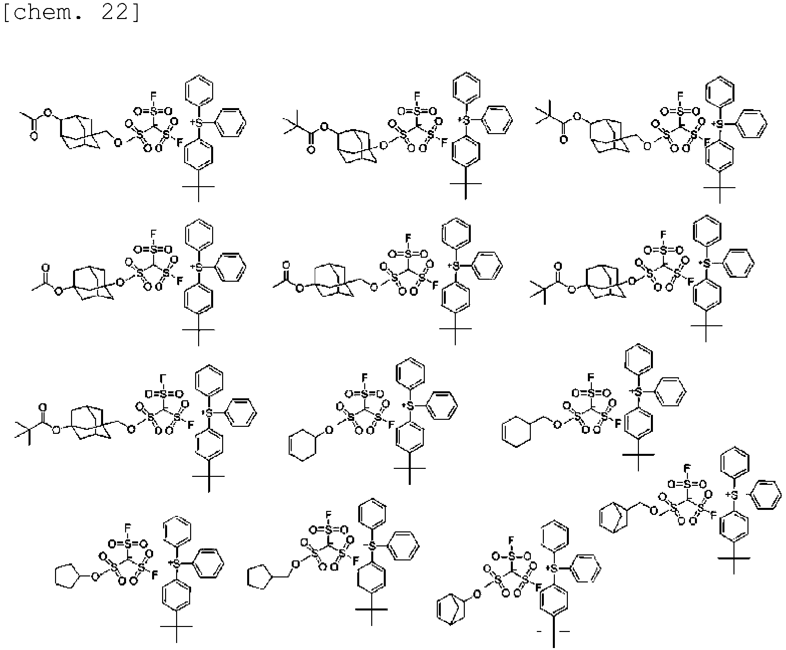

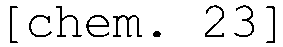

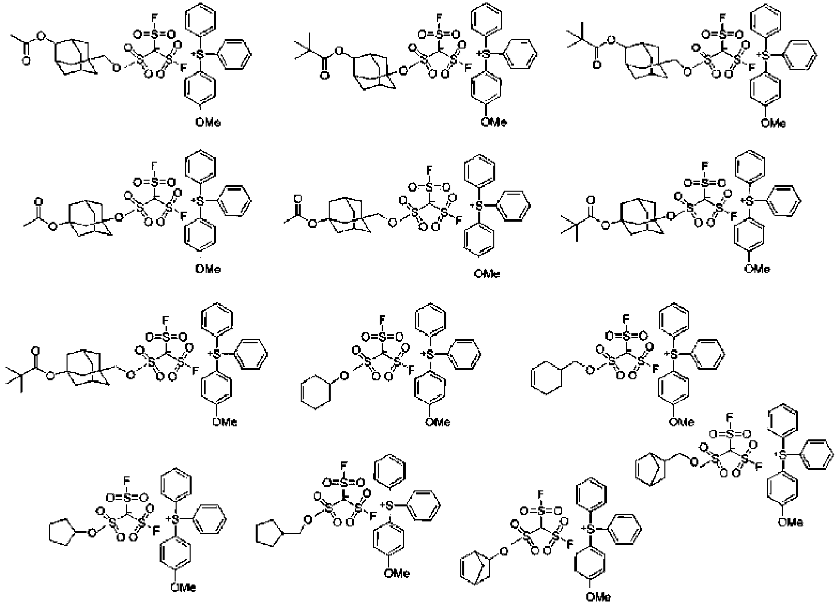

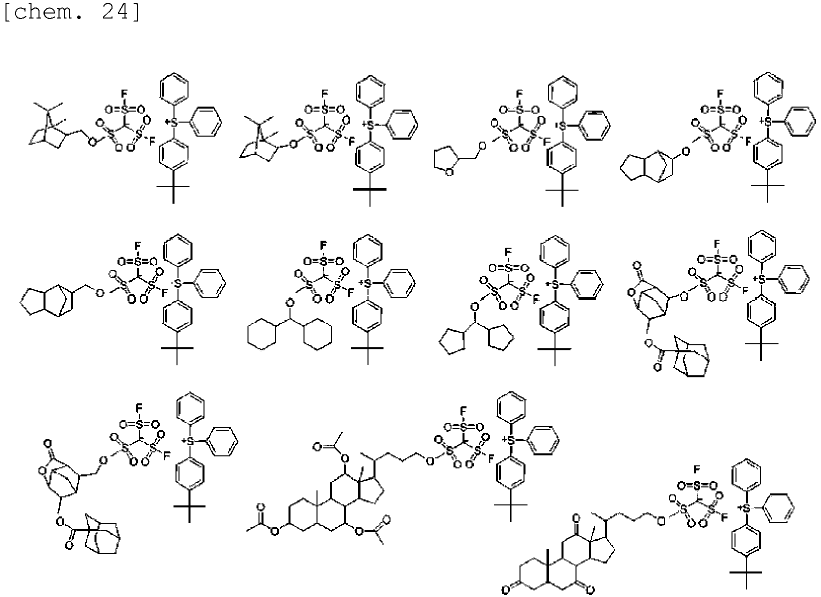

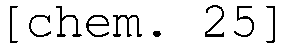

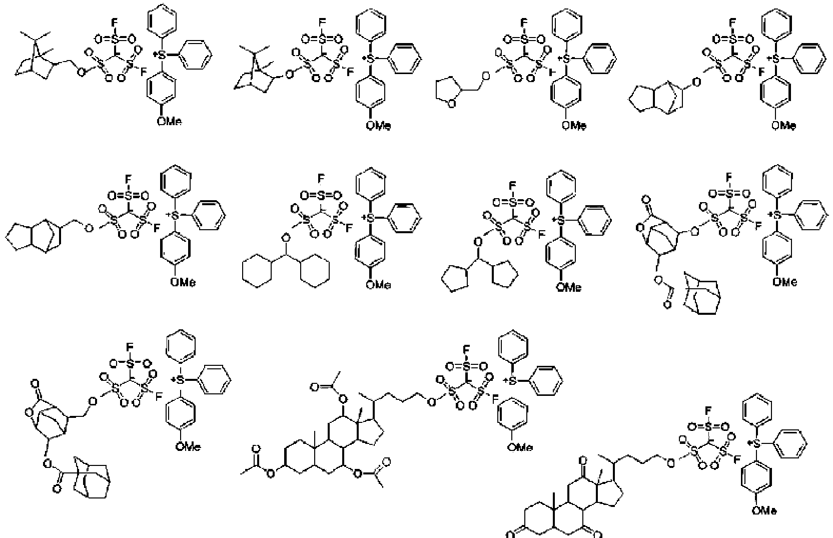

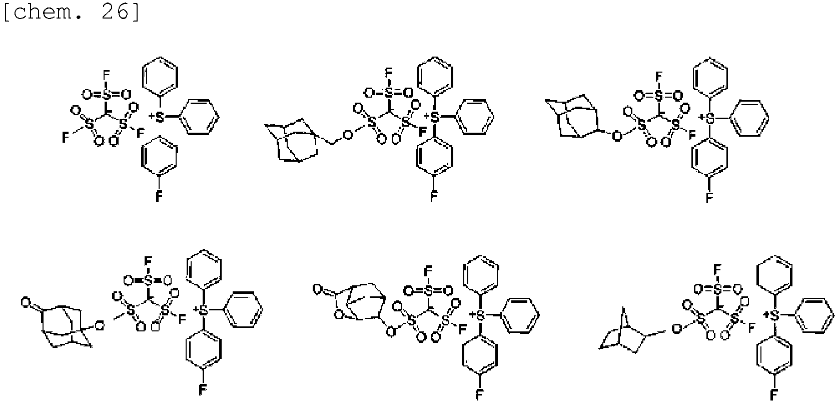

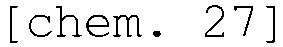

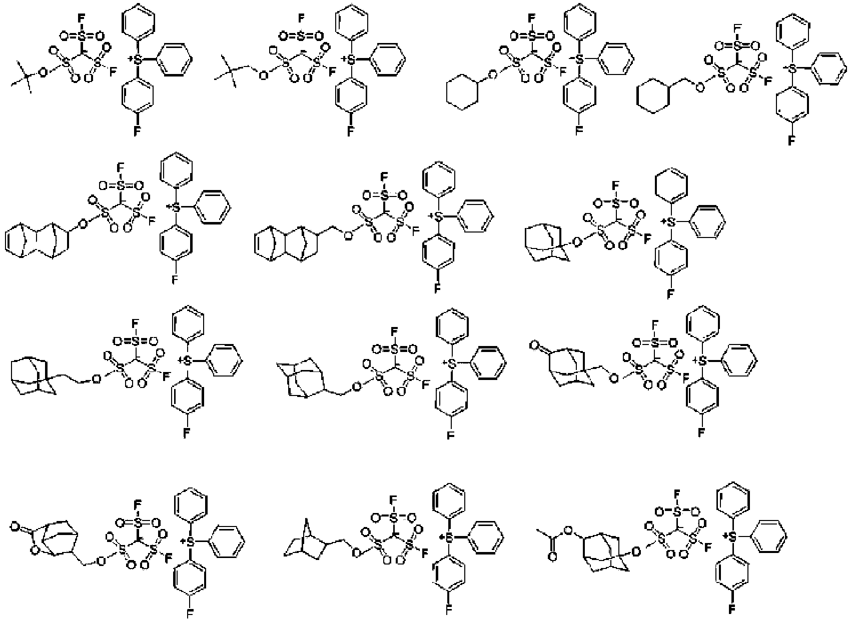

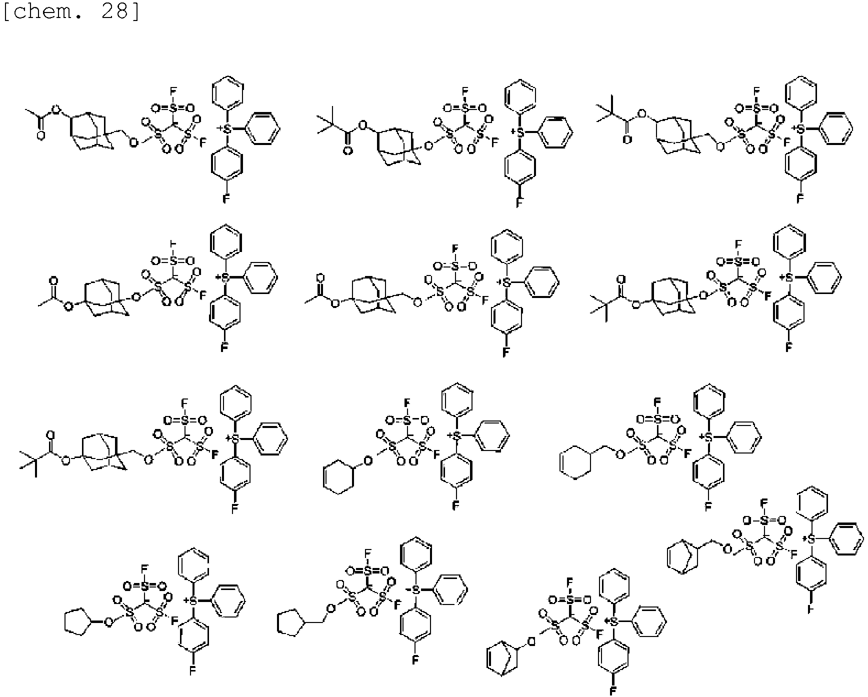

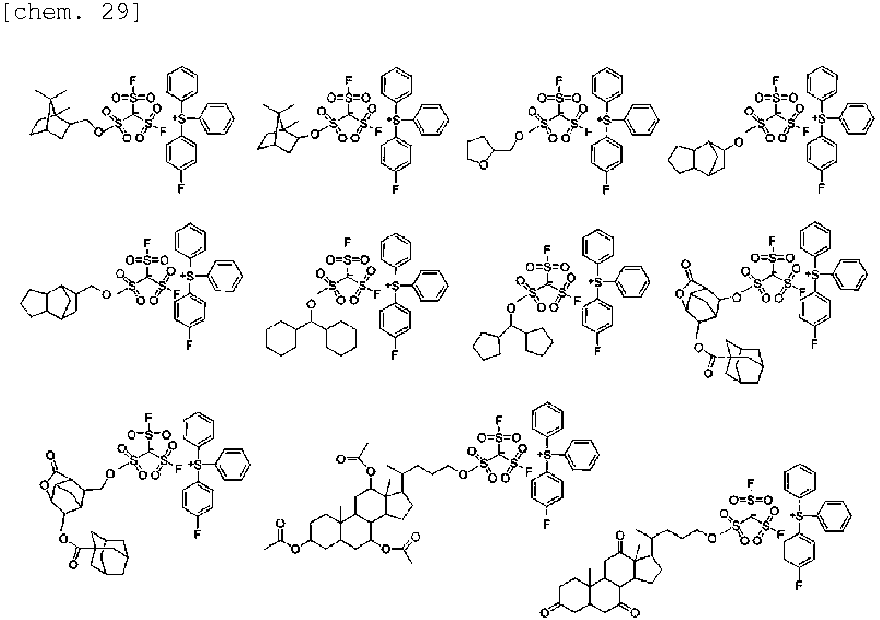

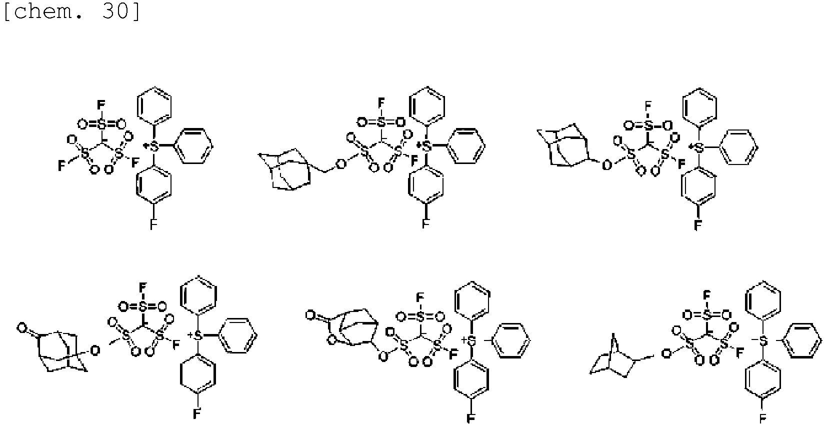

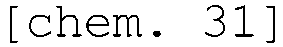

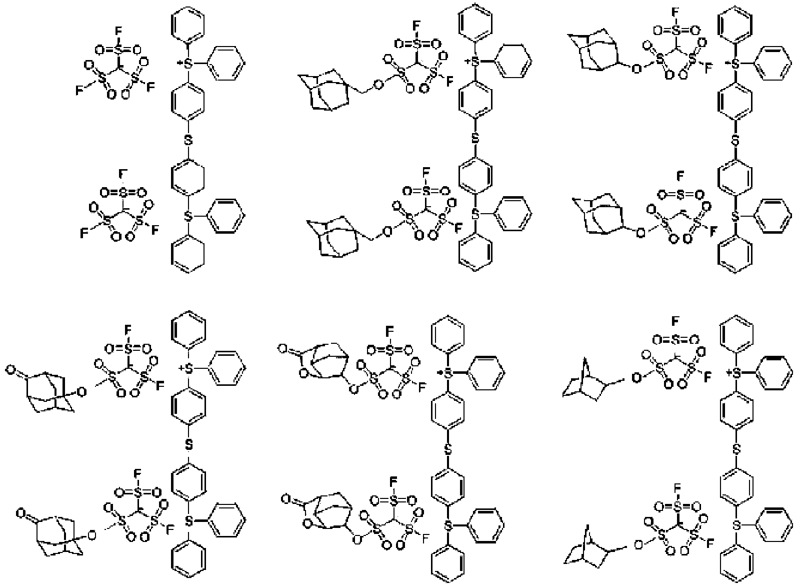

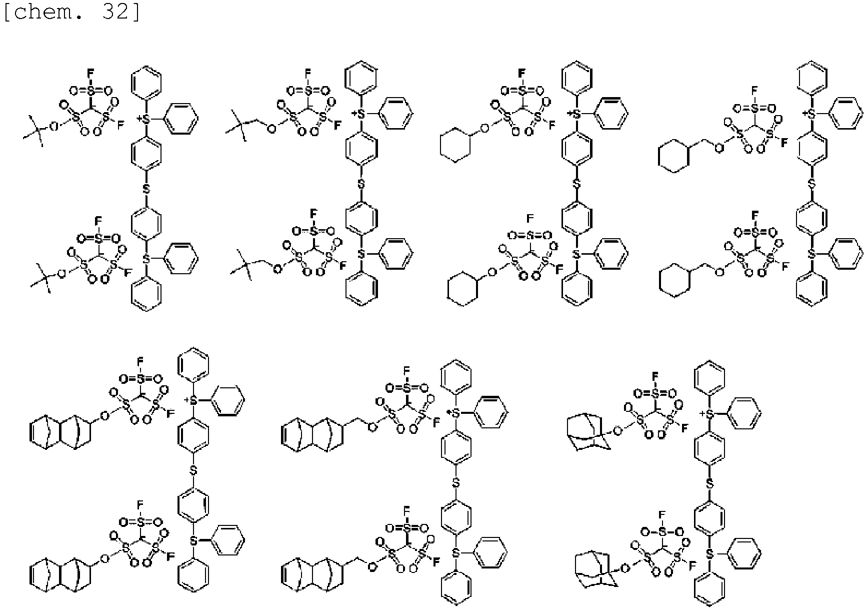

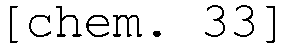

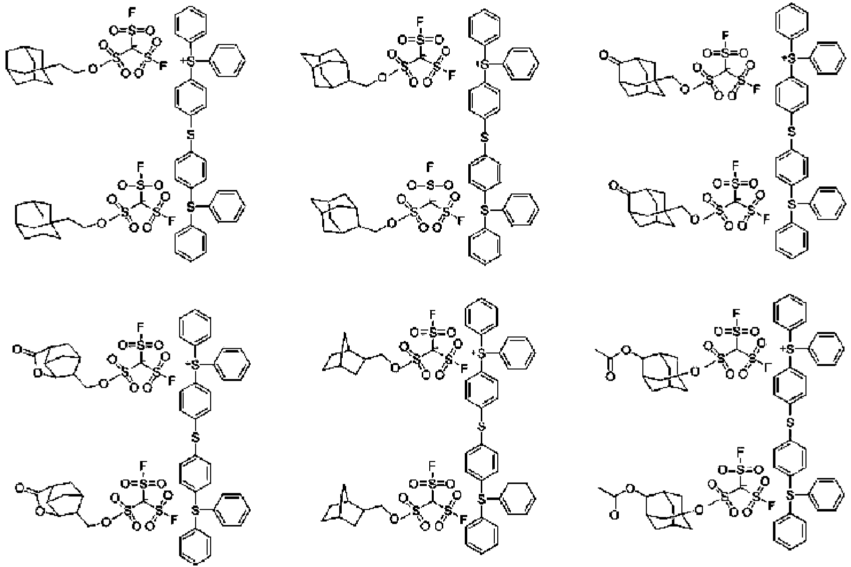

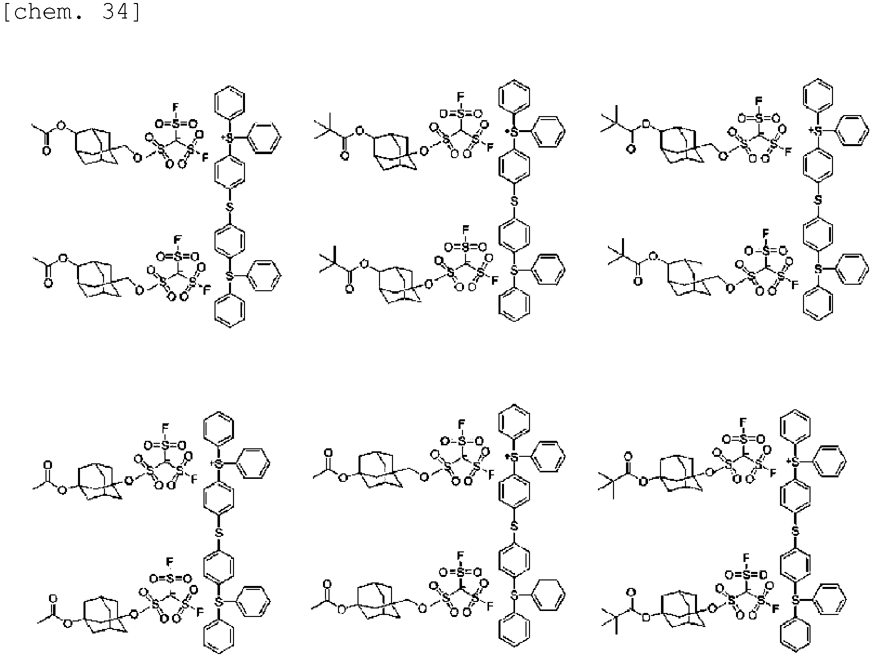

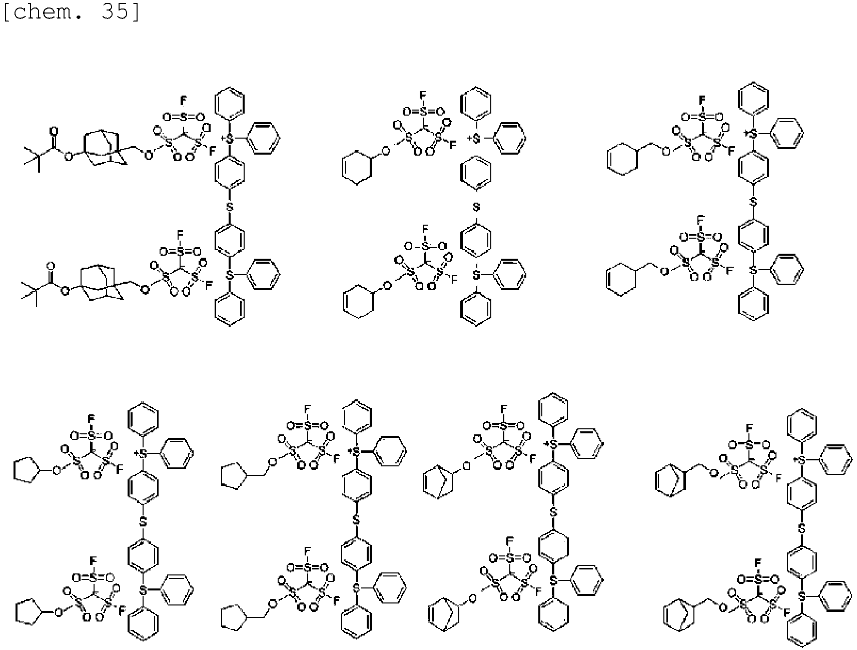

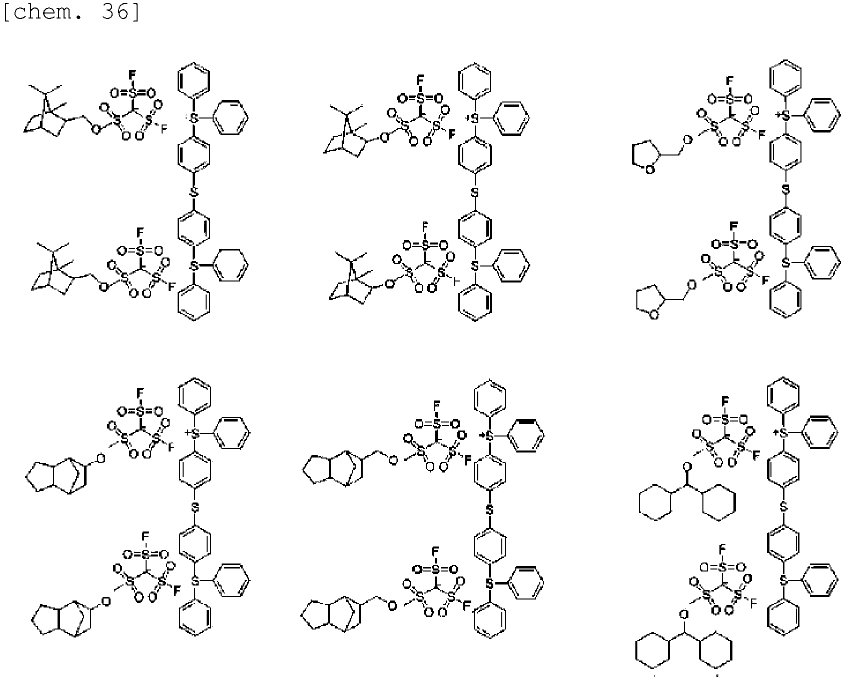

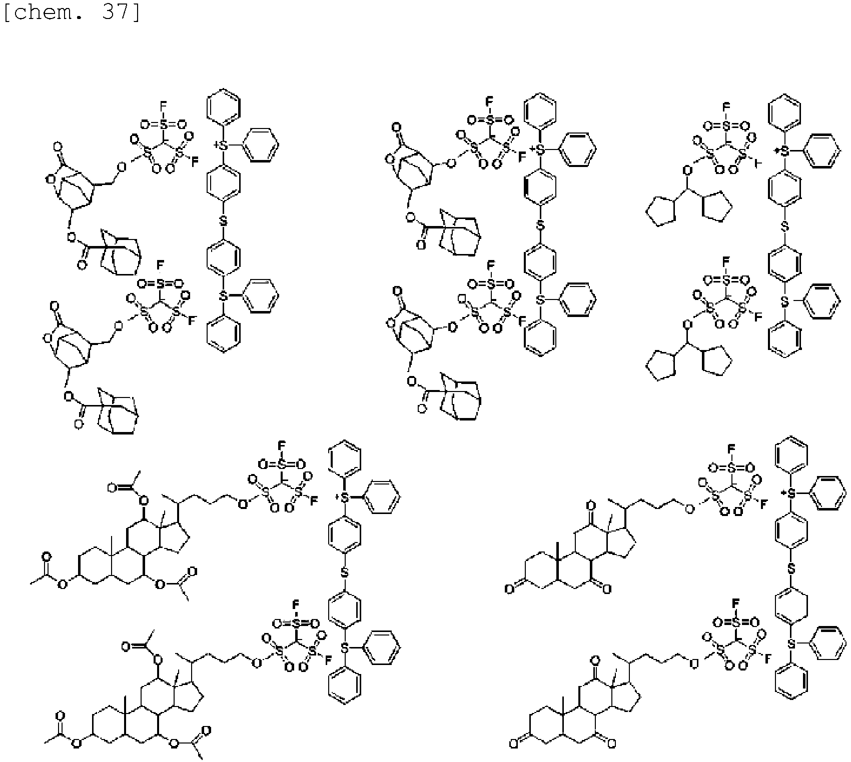

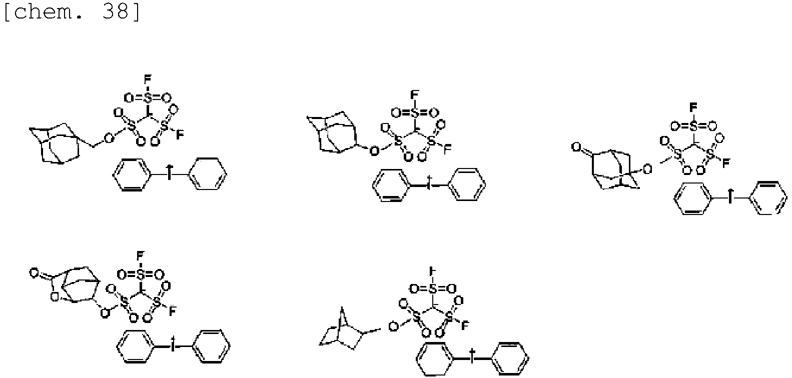

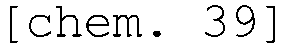

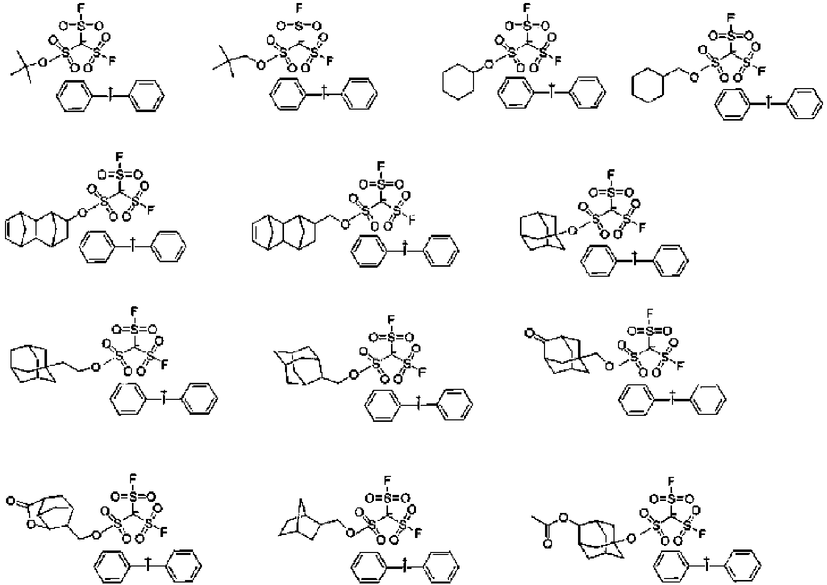

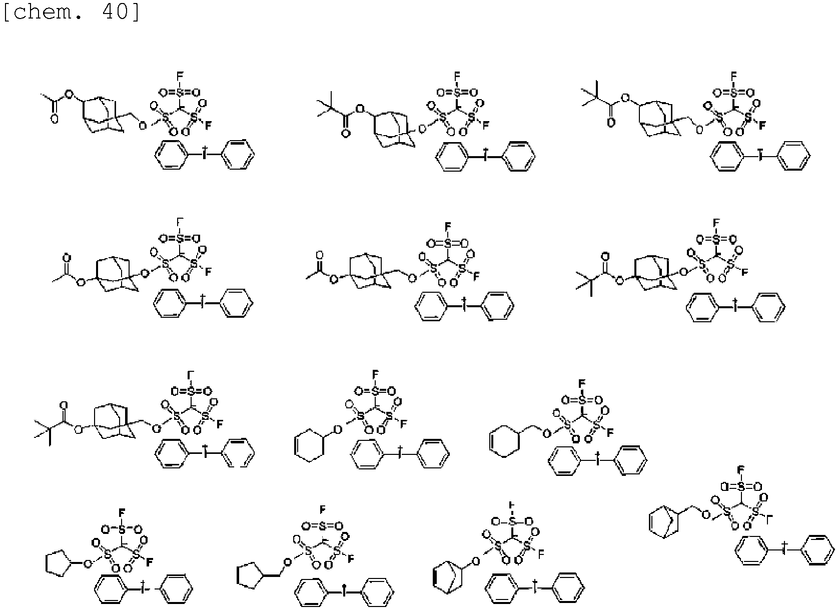

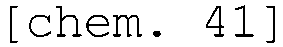

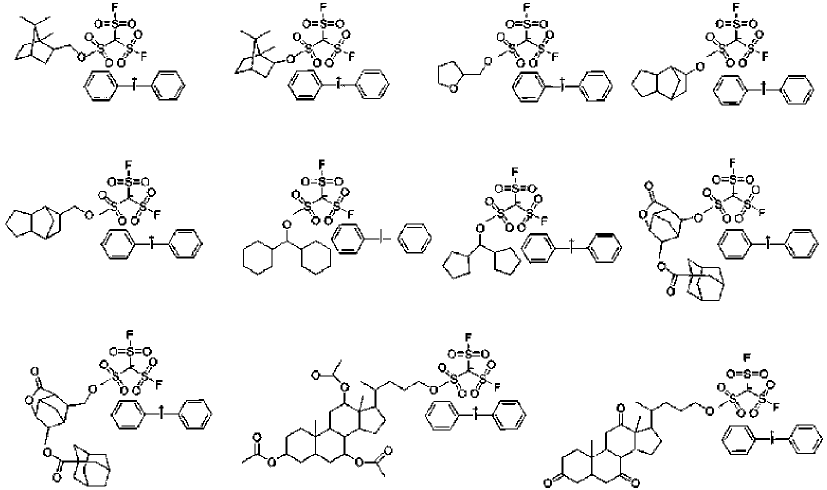

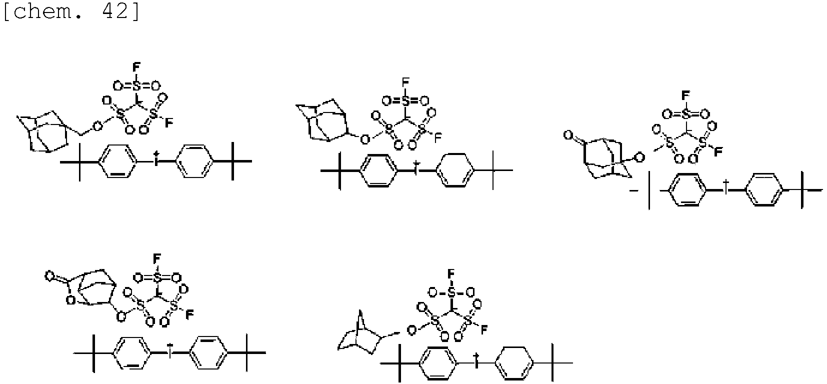

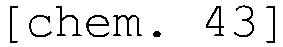

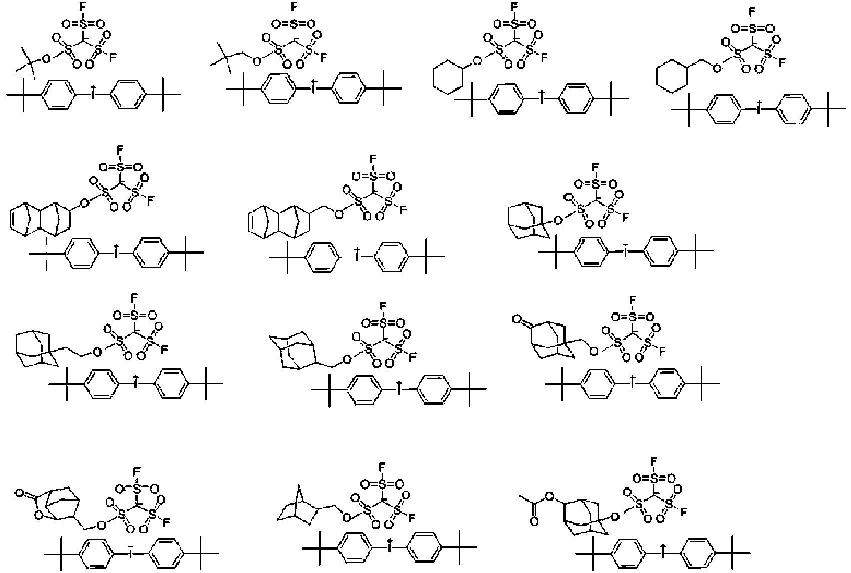

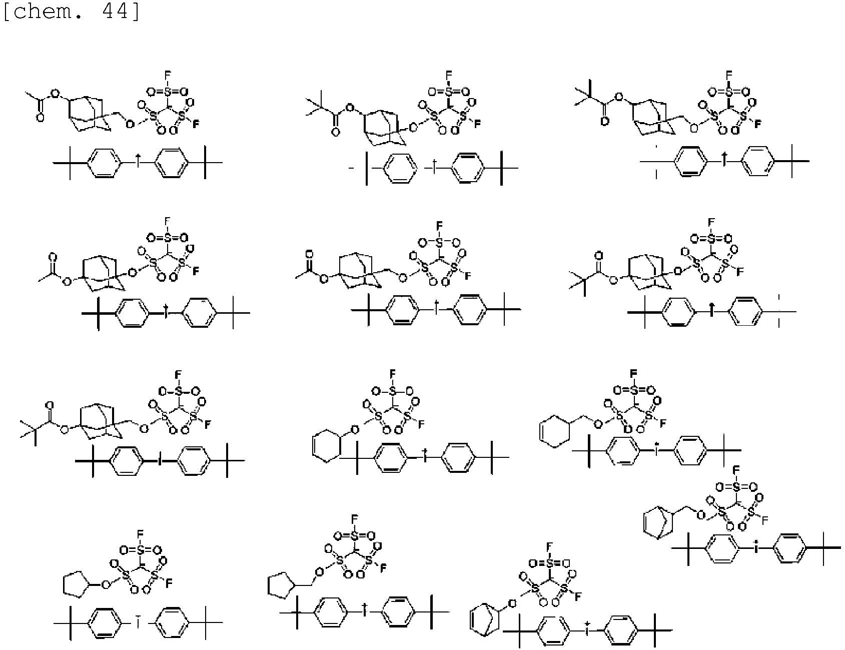

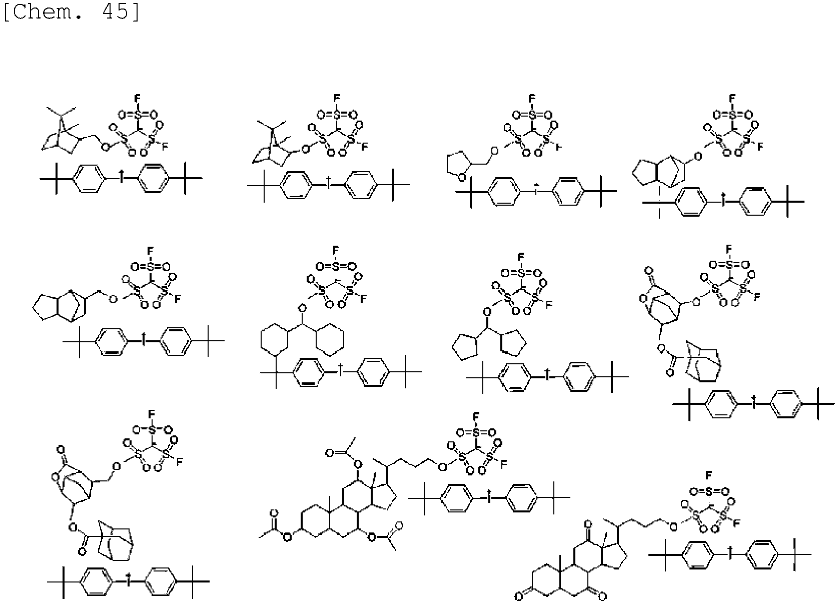

The radiation-sensitive resin composition may contain only one or two or more acid generators represented by General Formula (AG1).

The amount of the acid generator represented by General Formula (AG1) to be used may be appropriately adjusted in consideration of the sensitivity and other performances. The concentration of the acid generator represented by General Formula (AG1) in the non-volatile components in the radiation-sensitive resin composition is typically 1% to 50% by mass and preferably 2% to 40% by mass.

Hereinafter, a first embodiment and a second embodiment will be described as specific aspects of the radiation-sensitive resin composition.

### <Radiation-sensitive resin composition (first embodiment)>

The radiation-sensitive resin composition of the first embodiment contains an acid generator represented by General Formula (AG1) and a resin which is decomposed by the action of an acid to change the solubility in a developer.

Since the acid generator represented by General Formula (AG1) has already been described in detail, the description thereof will not be repeated.

### (Acid-decomposable resin)

The radiation-sensitive resin composition of the first embodiment contains a resin which is decomposed by the action of an acid to change the solubility in a developer. In the present specification, the "resin which is decomposed by the action of an acid to change the solubility in a developer" may be referred to as an "acid-decomposable resin".

Typically, the acid-decomposable resin has a group (hereinafter, also referred to as an "acid-decomposable group") which is decomposed by the action of an acid to generate a polar group such as an alkali-soluble group, in a main chain or a side chain of the resin or in both the main chain and the side chain. Typically, the acid-decomposable resin is insoluble or hardly soluble in a developer before being decomposed by the action of an acid.

The acid-decomposable group preferably has a structure in which a polar group such as an alkali-soluble group is protected by a group which is decomposed and eliminated by the action of an acid. Examples of the polar group include a phenolic hydroxy group, an alcoholic hydroxy group, a carboxyl group, a fluorinated alcohol group, a sulfonic acid group, a sulfonamide group, a sulfonylimide group, an (alkylsulfonyl) (alkylcarbonyl)methylene group, an (alkylsulfonyl) (alkylcarbonyl)imide group, a bis(alkylcarbonyl)methylene group, a bis(alkylcarbonyl)imide group, a bis(alkylsulfonyl)methylene group, a bis(alkylsulfonyl)imide group, a tris(alkylcarbonyl)methylene group, and a tris(alkylsulfonyl)methylene group. Preferred examples of the polar group include a phenolic hydroxyl group, a carboxyl group, and a fluorinated alcohol group.

A preferred group as the acid-decomposable group is a group in which a hydrogen atom of these polar groups (alkali-soluble groups and the like) is substituted with a group which is eliminated by an acid.

Examples of the group which is eliminated by an acid include -C(R³⁶)(R³⁷)(R³⁸), -C(R³⁶)(R³⁷)(OR³⁹), and -C(R⁰¹)(R⁰²)(OR³⁹). In these general formulae, R³⁶ to R³⁹ each independently represent an alkyl group, a monocyclic or polycyclic alicyclic group, an aryl group, an aralkyl group, or an alkenyl group. R³⁶ and R³⁷ may be bonded to each other to form a ring structure. R⁰¹ and R⁰² each independently represent a hydrogen atom, an alkyl group, a cycloalkyl group, an aryl group, an aralkyl group, or an alkenyl group.

In the pattern formation method described later, in a case where patterning is performed by light (wavelength: 193 nm) emitted from an ArF excimer laser, the acid-decomposable resin is preferably a resin which does not substantially include an aromatic structure such as a benzene ring. This is because the aromatic structure absorbs light having a wavelength of 193 nm. Here, the expression "does not substantially include an aromatic structure" means, for example, that the content of a structural unit having an aromatic structure is 5 mol% or less with respect to all structural units of the resin.

In a case where patterning is performed by light (wavelength: 193 nm) emitted from an ArF excimer laser, the acid-decomposable resin preferably includes a (meth)acrylic resin. From the viewpoint of etching resistance, the (meth)acrylic resin preferably includes an alicyclic skeleton.

The (meth)acrylic resin as the acid-decomposable resin preferably has (i) a structural unit having an acid-decomposable group, (ii) a structural unit having a lactone structure, (iii) a structural unit having a hydroxy group, and the like.

Examples of (i) the structural unit having an acid-decomposable group include a structural unit represented by General Formula (U1).

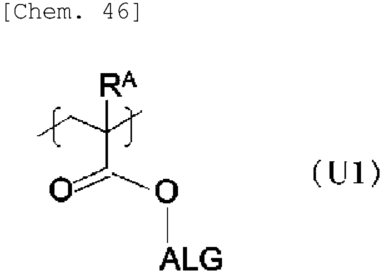

In General Formula (U1),
R^{A} is a hydrogen atom or a methyl group,
ALG is a group that is eliminated by an acid.

Preferred examples of the group of ALG that is eliminated by an acid include the group represented by -C(R³⁶)(R³⁷)(R³⁸) described above.

The ratio of the structural unit having an acid-decomposable group in the (meth)acrylic resin is, for example, 10 to 90 mol% and preferably 20 to 80 mol%. The (meth)acrylic resin may have a structural unit having two or more acid-decomposable groups.

Examples of (ii) the structural unit having a lactone structure include a structural unit represented by General Formula (U2).

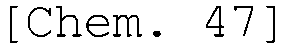

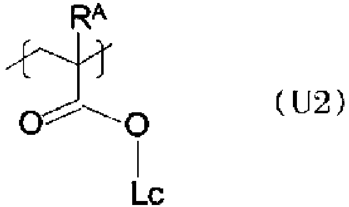

In General Formula (U2),
R^{A} is a hydrogen atom or a methyl group,
Lc is a group containing a lactone skeleton.

Examples of the lactone skeleton include a γ-butyrolactone skeleton and a norbornane lactone skeleton (norbornane 2,6-lactone skeleton).

The ratio of the structural unit having a lactone structure in the (meth)acrylic resin is, for example, 10 to 90 mol% and preferably 20 to 80 mol%. The (meth)acrylic resin may have a structural unit having two or more lactone structures.

Examples of (iii) the structural unit having a hydroxy group include a structural unit represented by General Formula (U3).

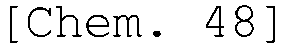

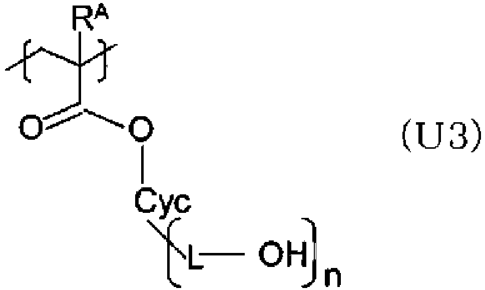

In General Formula (U3),
R^{A} is a hydrogen atom or a methyl group,
Cyc is a group in which (n+1) hydrogen atoms are removed from an alicyclic hydrocarbon,
L is a single bond or a divalent linking group,
n is 1 or 2.

Preferred examples of the alicyclic hydrocarbon constituting the skeleton of Cyc include adamantane and norbornane.

In a case where L is a divalent linking group, examples of L include a linear or branched alkylene group.

L may be fluorine-substituted. Specifically, L can be - C(CF₃)₂-. In a case where L is -C(CF₃)₂-, the hydrogen atom of - OH is easily dissociated. That is, in this case, -OH can function as an alkali-soluble group.

In a case where n is 2, two L's may be the same or different from each other.

The ratio of the structural unit having a hydroxy group in the (meth)acrylic resin is, for example, 10 to 90 mol%, and preferably 20 to 80 mol%. The (meth)acrylic resin may have a structural unit having two or more hydroxy groups.

The (meth)acrylic resin as the acid-decomposable resin may have or may not have other structural units in addition to (i) to (iii).

In addition to the (meth)acrylic resin, a polyhydroxystyrene-based resin can also be used as the acid-decomposable resin. The polyhydroxystyrene-based resin is preferably used in a case of performing patterning with light (wavelength: 248 nm) emitted from a KrF excimer laser or in a case of performing patterning with EUV light or electron beams.

Specific examples of the polyhydroxystyrene-based resin include (i) a resin having a hydroxystyrene structural unit and a hydroxystyrene structural unit protected by an acid-decomposable group, and (ii) a resin having a hydroxystyrene structural unit and a structural unit represented by General Formula (U1).

A calixarene-based resin can also be used as the acid-decomposable resin. The calixarene-based resin is also preferably used in a case of performing patterning with light (wavelength: 248 nm) emitted from a KrF excimer laser or in a case of performing patterning with EUV light or electron beams. Specific examples of the calixarene-based resin include (i) a resin having a phenolic hydroxy group structural unit and a phenolic hydroxy group structural unit protected by an acid-decomposable group.

The weight-average molecular weight of the acid-decomposable resin is, for example, 1,000 to 100,000, and preferably 2,000 to 50,000. The dispersity (weight-average molecular weight/number-average molecular weight) of the acid-decomposable resin is, for example, 1.0 to 2.5, and preferably 1.0 to 2.0.

The weight-average molecular weight and the dispersity of the acid-decomposable resin can be determined by gel permeation chromatography using polystyrene as a standard substance, for example.

The radiation-sensitive resin composition according to the first embodiment may contain only one acid-decomposable resin or two or more acid-decomposable resins.

The amount of the acid-decomposable resin in the non-volatile components of the radiation-sensitive resin composition is, for example, 30% to 99% by mass and preferably 50% to 95% by mass.

### (Quencher)

The radiation-sensitive resin composition according to the first embodiment preferably may contain a quencher, that is, a component having a function of deactivating an acid generated from the acid generator. By using the quencher, excessive movement of the acid in the resin film is suppressed, which leads to high resolution and reduction of line edge roughness.

The quencher is, for example, a basic compound and can be specifically a nitrogen-containing basic compound.

Specific examples of the quencher include guanidine, aminopyrrolidine, pyrazole, pyrazoline, piperazine, aminomorpholine, aminoalkylmorpholine, and piperidine. In addition, examples thereof include a compound having an imidazole structure, a compound having a diazabicyclo structure, a compound having an onium hydroxide structure, a compound having an onium carboxylate structure, a trialkylamine, an alkylamine derivative, and an aniline derivative. Furthermore, a compound known as a so-called "photodecomposable base" or "photodegradable base" in the field of photoresist can also be used as the quencher.

In a case where a quencher is used, only one quencher may be used, or two or more quenchers may be used.

In a case where a quencher is used, the amount thereof in the non-volatile components of the radiation-sensitive resin composition is, for example, 0.1% to 10% by mass and preferably 0.5% to 5% by mass.

### (Solvent)

The radiation-sensitive resin composition according to the first embodiment usually contains a solvent. In other words, the radiation-sensitive resin composition according to the first embodiment is usually a composition in which each of the above-described components is dissolved or dispersed in a solvent.

From the viewpoint of solubility and stability of the acid generator and the acid-decomposable resin, the solvent typically includes an organic solvent. From the same viewpoint, it is preferable that the solvent does not contain water. However, a trace amount of water that is unintentionally mixed in due to moisture in the air may be allowed.

Specific examples of the organic solvent include ketones, alcohols, polyhydric alcohols and derivatives thereof, ethers, esters, aromatic solvents, and fluorine-based solvents.

Examples of the ketones include acetone, methyl ethyl ketone, cyclopentanone, cyclohexanone, methyl isoamyl ketone, methyl isobutyl ketone, methyl isopentyl ketone, and 2-heptanone.

Examples of the alcohols include isopropanol, butanol, isobutanol, n-pentanol, isopentanol, tert-pentanol, 4-methyl-2-pentanol, 3-methyl-3-pentanol, 2,3-dimethyl-2-pentanol, n-hexanol, n-heptanol, 2-heptanol, n-octanol, n-decanol, s-amyl alcohol, t-amyl alcohol, isoamyl alcohol, 2-ethyl-1-butanol, lauryl alcohol, hexyldecanol, and oleyl alcohol.

Examples of the polyhydric alcohols and derivatives thereof include ethylene glycol, ethylene glycol monoacetate, ethylene glycol dimethyl ether, diethylene glycol, diethylene glycol dimethyl ether, diethylene glycol monoacetate, propylene glycol, propylene glycol monoacetate, propylene glycol monomethyl ether (PGME), propylene glycol monoethyl ether, propylene glycol monopropyl ether, propylene glycol monobutyl ether, propylene glycol monomethyl ether acetate (PGMEA), dimethyl ether, monomethyl ether, monoethyl ether, monopropyl ether, monobutyl ether, and monophenyl ether of dipropylene glycol or dipropylene glycol monoacetate.

Examples of the ethers include diethyl ether, diisopropyl ether, tetrahydrofuran, dioxane, and anisole.

Examples of the esters include methyl lactate, ethyl lactate (EL), methyl acetate, ethyl acetate, butyl acetate, methyl pyruvate, ethyl pyruvate, methyl methoxypropionate, ethyl ethoxypropionate, and γ-butyrolactone.

Examples of the aromatic solvent include xylene and toluene.

Examples of the fluorine-based solvent include freon, alternative freon, perfluoro compounds, and hexafluoroisopropyl alcohol.

In a case where a solvent is used, only one solvent may be used, or two or more solvents may be used.

From the viewpoint of providing a film having a desired thickness, the solvent can be used in an amount such that the concentration of non-volatile components is, for example, 1% to 60% by mass and preferably 5% to 50% by mass.

### (Other optional components)

The radiation-sensitive resin composition according to the first embodiment may contain or may not contain optional components other than those described above.

Examples of the optional components include an acid generator other than the acid generator represented by General Formula (AG1), a surfactant (preferably a fluorine-based surfactant), an antioxidant, a dye, a plasticizer, a photosensitizer, a light absorber, and a compound that promotes solubility in a developer (for example, a low-molecular-weight phenolic compound or a carboxylic acid).

In addition, in a case where the radiation-sensitive resin composition according to the first embodiment is applied to immersion exposure, a water-repellent polymer may be used. As a preferred water-repellent polymer, a fluorine-containing polymer or a silicon-containing polymer is known.

### <Radiation-sensitive resin composition (second embodiment)>

The radiation-sensitive resin composition according to the second embodiment contains an acid generator represented by General Formula (AG1) and a component which is increased in molecular weight by the action of an acid and whose solubility in a developer is changed.

In the radiation-sensitive resin composition according to the second embodiment, specific aspects, preferred amounts of use, and the like of the acid generator represented by General Formula (AG1) are as described in the section of <Radiation-sensitive resin composition>.

As the component which is increased in molecular weight by the action of an acid and whose solubility in a developer is changed, a combination of an alkali-soluble resin and a crosslinking agent is usually considered. The alkali-soluble resin is a resin which is soluble in an alkaline aqueous solution as a developer described later and is neither poorly soluble nor insoluble.

Examples of the alkali-soluble resin include a novolac resin, a hydrogenated novolac resin, an acetone-pyrogallol resin, o-polyhydroxystyrene, m-polyhydroxystyrene, p-polyhydroxystyrene, hydrogenated polyhydroxystyrene, halogen- or alkyl-substituted polyhydroxystyrene, a hydroxystyrene-N-substituted maleimide copolymer, an o/p- and m/p-hydroxystyrene copolymer, a partially O-alkylated product (for example, 5 to 30 mol% of an O-methylated product, an O-(1-methoxy)ethylated product, an O-(1-ethoxy)ethylated product, an O-2-tetrahydropyranylated product, an O-(t-butoxycarbonyl)methylated product, and the like) or an O-acylated product (for example, 5 to 30 mol% of an o-acetylated product, an O-(t-butoxy)carbonylated product, and the like) with respect to a hydroxyl group of polyhydroxystyrene, a styrene-maleic anhydride copolymer, a styrene-hydroxystyrene copolymer, an α-methylstyrene-hydroxystyrene copolymer, a carboxyl group-containing methacrylic resin and a derivative thereof, and a polyvinyl alcohol derivative.

A particularly preferred alkali-soluble resin is a novolac resin, o-polyhydroxystyrene, m-polyhydroxystyrene, p-polyhydroxystyrene, alkyl-substituted polyhydroxystyrene, a partially O-alkylated or O-acylated product of polyhydroxystyrene, a styrene-hydroxystyrene copolymer, or an α-methylstyrene-hydroxystyrene copolymer.

In a case where an alkali-soluble resin is used, only one alkali-soluble resin may be used, or two or more alkali-soluble resins may be used.

The crosslinking agent is not particularly limited as long as it is a compound which crosslinks the alkali-soluble resin by the action of an acid. Specifically, the following (1) to (3) can be preferably mentioned. Specific compounds corresponding to these are described in, for example, paragraphs 0208 and 0209 of JP2004-117688A.
(1) Hydroxymethyl, alkoxymethyl, and acyloxymethyl derivatives of a phenol derivative
(2) Compound having an N-hydroxymethyl group, an N-alkoxymethyl group, or an N-acyloxymethyl group
(3) Compound having an epoxy group

Incidentally, in a case where the crosslinking agent has an alkoxymethyl group, the number of carbon atoms in the alkoxymethyl group is preferably 6 or less. In addition, in a case where the crosslinking agent has an acyloxymethyl group, the number of carbon atoms in the acyloxymethyl group is preferably 6 or less.

In a case where a crosslinking agent is used, only one crosslinking agent may be used, or two or more crosslinking agents may be used.

In consideration of the balance of various performances, the ratio between the alkali-soluble resin and the crosslinking agent may be appropriately adjusted. The amount of the crosslinking agent may be, for example, 5 to 50 parts by mass, and specifically 10 to 40 parts by mass with respect to 100 parts by mass of the alkali-soluble resin.

The ratio of the component (typically, the alkali-soluble resin and the crosslinking agent) whose solubility in a developer is changed by increasing the molecular weight by the action of an acid in the non-volatile components of the radiation-sensitive resin composition according to the second embodiment is, for example, 50% to 95% by mass and preferably 60% to 90% by mass.

The radiation-sensitive resin composition according to the second embodiment can contain various additive components and solvents in addition to the acid generator represented by General Formula (AG1) and the component whose solubility in a developer is changed by increasing the molecular weight by the action of an acid. Examples of the additive component include the quencher and other optional components described in the section of <Radiation-Sensitive Resin Composition (First Embodiment)> above. Specific aspects and amounts of the additive components and solvents to be used are as described in the section of <Radiation-Sensitive Resin Composition (First Embodiment)> above.

### <Pattern Formation Method and Method for Manufacturing Electronic Device>

(Hereinafter, unless otherwise specified, the radiation-sensitive resin composition according to the first embodiment and the radiation-sensitive resin composition according to the second embodiment will be collectively referred to as "radiation-sensitive resin composition").

A pattern can be formed using the radiation-sensitive resin composition. Specifically, it is possible to form a pattern by
· forming a film using a radiation-sensitive resin composition,
·irradiating the film with radiation,
·developing the film irradiated with the radiation.

In addition, an electronic device can be manufactured by using the formed pattern as a mask in dry etching, ion implantation, metal sputtering, or metal plating.

The film formation, the irradiation with radiation, and the development will be briefly described below. In addition, optional or additional matters other than these will be described.

### (Film Formation)

The substrate on which the film is formed is not particularly limited. Examples thereof include a glass substrate, a silicon wafer, a ceramic substrate, an aluminum substrate, a SiC wafer, a GaN wafer, a copper substrate, and a copper-plated substrate. The substrate may be an unprocessed substrate or a substrate on which an electrode or an element is formed on the surface. From the viewpoint of suppressing deterioration of the resolution due to reflection of the irradiated light on the substrate, it is preferable that an antireflective film is provided on the surface of the substrate.

The method of forming the film is not particularly limited. In the field of manufacturing electronic devices, spin coating using a spinner is generally used, but other methods may be used. For example, spray coating using a spray coater, immersion, printing, roll coating, an inkjet method, or the like may be used.

The drying of the radiation-sensitive resin composition applied onto the substrate is typically performed by a heat treatment. The heating temperature is usually 80°C to 250°C and preferably 90°C to 220°C. In addition, the heating time varies depending on the heating device, but in a case where a hot plate is used, the heating time is usually 30 to 300 seconds and preferably about 40 to 180 seconds, and in a case where a hot air oven is used, the heating time is usually 5 to 60 minutes and preferably about 10 to 30 minutes.

The thickness of the film is not particularly limited, and may be appropriately adjusted according to the application to be applied, the pattern to be finally obtained, and the like. The thickness of the film is, for example, 5 to 500 nm, preferably 10 to 400 nm, and more preferably 15 to 300 nm.
The thickness of the film can be adjusted by adjusting the non-volatile component concentration of the radiation-sensitive resin composition, changing the coating method or the coating conditions, and the like.

### (Irradiation with radiation)

The irradiation with radiation is usually performed by applying radiation to the film through an appropriate photomask. In a case where the beam diameter is small, such as in the case of an electron beam, a photomask may not be used.

The type of radiation is as described above. In the manufacture of electronic devices, light having a wavelength of 248 nm emitted from a KrF excimer laser, light having a wavelength of 193 nm emitted from an ArF excimer laser, extreme ultraviolet light (EUV light) having a wavelength of 13.5 nm, electron beams, and the like are usually used.

In particular, in a case where the film is irradiated with light having a wavelength of 193 nm emitted from an ArF excimer laser, immersion exposure can also be applied in order to increase the resolution. In this case, a top coat film may be provided for water repellency.

Examples of the device for irradiation with radiation include a contact aligner, a mirror projection, a stepper, and a scanner.

The irradiation amount of radiation may be appropriately adjusted depending on the amount of the acid generator in the film and the like, and is, for example, about 1 to 500 mJ/cm².

The film can also be heated after the exposure and before the development step as necessary (post exposure bake (PEB)). The temperature is, for example, 70°C to 150°C, and preferably 80°C to 140°C. In addition, the time is usually 30 to 300 seconds and preferably 40 to 180 seconds, for example, in a case where a hot plate is used. By performing PEB, the solubilization or insolubilization reaction of the resin due to the acid generated from the acid generator can be further promoted, and thus it is possible to expect further increase in sensitivity.

### (Development)

A pattern can be obtained by developing the film irradiated with radiation with a developer. The development can be performed by, for example, a method such as an immersion method, a puddle method, or a rotary spray method.

As the developer, an alkaline aqueous solution is usually used. Specific examples of the alkaline aqueous solution include (i) an inorganic alkaline aqueous solution such as sodium hydroxide, sodium carbonate, sodium silicate, or ammonia, (ii) an organic amine aqueous solution such as ethylamine, diethylamine, triethylamine, or triethanolamine, and (iii) an aqueous solution of a quaternary ammonium salt such as tetramethylammonium hydroxide or tetrabutylammonium hydroxide.

As the developer, in particular, a tetramethylammonium hydroxide aqueous solution is preferable. The concentration of the tetramethylammonium hydroxide is preferably 0.1% to 10% by mass and more preferably 0.5% to 5% by mass.

A film is formed using the radiation-sensitive resin composition according to the first embodiment, and the film is irradiated with radiation and then subjected to a development treatment using an alkaline aqueous solution, thereby usually obtaining a positive pattern.

A film is formed using the radiation-sensitive resin composition according to the second embodiment, and the film is irradiated with radiation and then subjected to a development treatment using an alkaline aqueous solution, thereby usually obtaining a negative pattern.

On the other hand, as the developer, an organic solvent-based developer (a developer in which an organic solvent accounts for 90% by mass or more) can also be used instead of the alkaline aqueous solution.

In a case where a film is formed using the radiation-sensitive resin composition according to the first embodiment, and the film is irradiated with radiation and then subjected to a development treatment using an organic solvent-based developer, a negative pattern can be usually obtained instead of a positive pattern. This is because the alkaline aqueous solution and the organic solvent-based developer have opposite polarities.

In a case where a film is formed using the radiation-sensitive resin composition according to the second embodiment, and the film is irradiated with radiation and then subjected to a development treatment using an organic solvent-based developer, a negative pattern can be usually obtained, as in the development using an alkaline aqueous solution. Since the mechanism of insolubilization in the developer is an increase in molecular weight, a negative pattern is usually formed regardless of whether the developer is an alkaline aqueous solution or an organic solvent-based developer.

The development treatment using an organic solvent-based developer is described in, for example, JP2008-292975A. As the organic solvent-based developer, a butyl acetate developer is particularly widely known.

A pattern can be formed by development, but it is preferable to wash the pattern and the substrate with a rinse liquid after the development.

In a case where an alkaline aqueous solution is used as the developer, ultrapure water is suitable as the rinse liquid.

In a case where an organic solvent-based developer is used as the developer, an organic solvent such as an alcohol-based solvent is suitable as the rinse liquid.

The obtained pattern can be used, for example, as a mask during dry etching, a mask in an ion implantation process, or a mask for metal sputtering or metal plating in the manufacture of electronic devices.

### <Compound, preferably acid generator>

Hereinbefore, the embodiments of the novel technology by the present inventors have been described from the viewpoint of the "composition", the pattern formation method using the composition, and the method for manufacturing an electronic device.

It should be noted that the novel technology by the present inventors can be regarded not only as a composition but also as a compound, preferably an acid generator. That is, the compound (preferably, the acid generator) represented by General Formula (AG1) described above is novel and can be useful.

In a case where the compound represented by General Formula (AG1) described above is an acid generator, an acid can be generated by applying an external stimulus to the compound (for example, irradiating the compound with radiation).

Since the description of the compound represented by General Formula (AG1), preferably the acid generator, has been sufficiently described in the section of <Radiation-sensitive resin composition>, the description thereof will not be repeated.

### <Method for producing compound, preferably acid generator or precursor thereof>

The compound (preferably, the acid generator or the precursor thereof) represented by General Formula (AG1) can be produced (synthesized) based on a known technique or method in organic synthetic chemistry. For specific examples of the production (synthesis) conditions and raw materials, refer to the synthesis examples described later.

Incidentally, examples of the "precursor" of the acid generator represented by General Formula (AG1) include a compound having substantially no sensitivity to radiation, for example, a compound in which a cation moiety is a metal cation.

The procedure for production (synthesis) will be briefly described. The compound represented by General Formula (AG1) can be produced (synthesized) by the following procedure, for example. In order to prevent unintended decomposition by radiation, it is preferable that at least a part of the following procedure is performed under conditions in which illumination is appropriately controlled.
(i) Fuming sulfuric acid and acetic anhydride are reacted with each other to synthesize methanetrissulfonic acid.
(ii-1) A fluorinating agent (preferably, a nucleophilic fluorinating agent) is reacted with the methanetrisulfonic acid, and then a metal fluoride such as potassium fluoride is reacted therewith. In this manner, a compound (precursor of the acid generator) represented by General Formula (M-FSM) can be obtained.

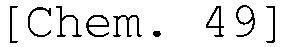

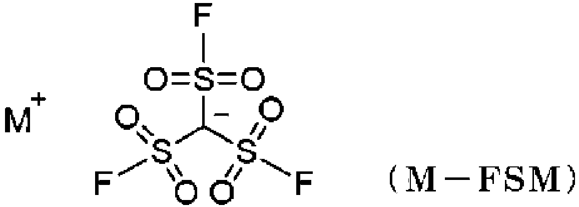

In General Formula (M-FSM), M⁺ is a metal cation.

The metal cation can be K⁺, Na⁺, or the like, but is not particularly limited as long as the compound represented by General Formula (M-FSM) is electrically neutral.

The fluorinating agent is not particularly limited, and preferred examples thereof include FLUOLEAD (product name, compound name: 4-tert-butyl-2,6-dimethylphenylsulfur trifluoride) manufactured by UBE Corporation. Of course, other known fluorinating agents may be used as long as the compound represented by General Formula (M-FSM) can be obtained. The amount of the fluorinating agent used can be preferably 3.0 to 8.0 equivalents.

Examples of the metal fluoride include sodium fluoride in addition to the above-described potassium fluoride. Of course, the metal fluoride is not particularly limited as long as the compound represented by General Formula (M-FSM) can be obtained. The amount of the metal fluoride used can be preferably 3.0 to 20.0 equivalents. Alternatively, instead of the metal fluoride, it may be possible to use anhydrous hydrogen fluoride (AHF) or an organic hydrogen fluoride salt such as hydrogen fluoride-pyridine or triethylamine trihydrogen fluoride.

The reaction temperature here can be, for example, 10°C to 50°C, and the reaction time can be, for example, 2 to 24 hours.

Preferred examples of the solvent here include a halogen-based solvent such as dichloromethane or dichloroethane. In addition, it is considered that heptane, toluene, tetrahydrofuran, acetonitrile, or the like can also be used. Of course, solvents not mentioned here can also be used as long as the compound represented by General Formula (M-FSM) can be obtained.
(ii-2) In a case where a compound in which at least any one of R¹ or R² is -OR in the compound represented by General Formula (AG1) is finally desired to be produced, the compound represented by General Formula (M-FSM) is reacted with a compound represented by R-OH. In this manner, in the compound represented by General Formula (ag1), a compound in which at least one of R¹ or R² is -OR (a precursor of the acid generator) can be obtained.

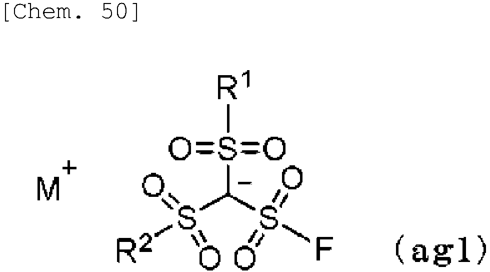

In the General Formula (ag1),
M⁺ is a metal cation,
R¹ and R² are each independently a fluorine atom or -OR, and R is a monovalent organic group.

The metal cation can be K⁺, Na⁺, or the like, but is not particularly limited as long as the compound represented by General Formula (M-FSM) is electrically neutral.

Specific examples and preferred aspects of the monovalent organic group of R are as described in the section of <Radiation-sensitive resin composition (first embodiment)>.
(iii) The metal cation M⁺ in the compound represented by General Formula (M-FSM) or the compound represented by General Formula (ag1) is replaced with an appropriate cation. In this manner, a compound represented by General Formula (AG1) (preferably, an acid generator) can be obtained.

As a specific example, a compound represented by General Formula (AG1-1) (preferably, an acid generator) can be produced by reacting the compound represented by General Formula (ag1) with a compound represented by General Formula A₁⁺X⁻. In General Formula A₁⁺X⁻, A₁⁺ is a sulfonium cation or an iodonium cation, and X⁻ is a halide ion (preferably Cl⁻, Br⁻, or I⁻), a sulfonate ion, a sulfate ion, a phosphate ion, a hydroxide ion, or a carboxylate ion.

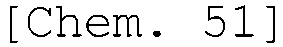

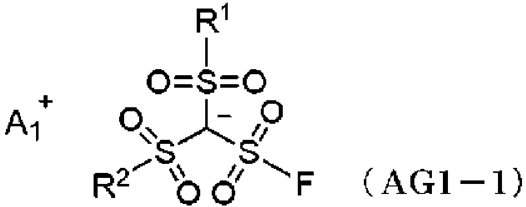

In the General Formula (AG1-1),
A₁⁺ is a sulfonium cation or an iodonium cation,
R¹ and R² are each independently a fluorine atom or -OR, and R is a monovalent organic group.

Specific examples and preferred aspects of the sulfonium cation or the iodonium cation as A₁⁺ are as described in the section of <Radiation-sensitive resin composition>. The same applies to the structure of the sulfonium moiety in the sulfonium chloride and the structure of the iodonium moiety in the iodonium chloride.

Specific examples and preferred aspects of the monovalent organic group of R are as described in the section of <Radiation-sensitive resin composition>.

Although the embodiments of the present invention have been described above, these are examples of the present invention, and various configurations other than the above can be adopted. In addition, the present invention is not limited to the embodiments described above and modifications, improvements, and the like are included in the present invention in a range in which it is possible to achieve the object of the present invention.

### Examples

A detailed description will be given of embodiments of the present invention based on Examples and Comparative Examples. It should be noted that the present invention is not limited to Examples only.

### <Synthesis Example>

### [Synthesis Example 1: Synthesis of Methanetrisulfonic Acid]

30% fuming sulfuric acid (manufactured by FUJIFILM Wako Pure Chemical Corporation, 137 g, 41 g of SO₃ in fuming sulfuric acid, 512 mmol, 5.2 equivalents) was placed in a 0.5 L three-necked eggplant flask and cooled in an ice bath.

Acetic anhydride (manufactured by FUJIFILM Wako Pure Chemical Corporation, 10 g, 98.3 mmol) was added to the flask. Thereafter, the temperature was raised stepwise, and the mixture was stirred overnight at 90°C.

After the reaction solution was cooled to room temperature, vacuum filtration was performed using a polytetrafluoroethylene filter having a pore size of 1.0 µm, thereby obtaining 56.7 g of a filter residue and 77.9 g of a filtrate. As a result of quantifying the filter residue by ¹H-NMR, the target product was contained in an amount of 118 mmol (quantitative yield: 70%, internal standard substance: 1,4-bis-trifluoromethylbenzene).

The obtained filter residue was subjected to dispersion washing sequentially with toluene (manufactured by FUJIFILM Wako Pure Chemical Corporation, 111 g × 2 times) and acetonitrile (manufactured by FUJIFILM Wako Pure Chemical Corporation, 81 g). Then, the obtained crystals were dried under the conditions of a degree of vacuum of < 2 kPa and a bath temperature of 50°C. The "degree of vacuum < 2 kPa" means that the absolute pressure was set to less than 2 kPa (the same applies hereinafter to the same description).

In this manner, methanetrisulfonic acid (14.9 g, quantitative value: 42.5 mmol, purity: 66% by mass, yield: 32%) was obtained.

The ¹H-NMR analysis results of the obtained methanetrisulfonic acid are shown below.

¹H-NMR (DMSO, standard substance: DMSO): 4.70 ppm (s, 1H, C-H)

For reference, a scheme diagram of the above-described reaction is shown below.

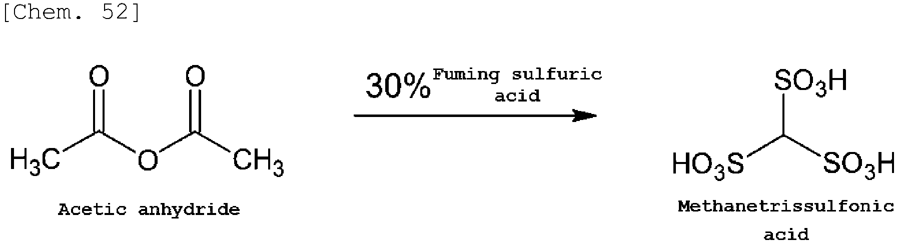

### [Synthesis Example 2: Synthesis of K-FSM] (Chemical structure of K-FSM is shown below)

Methanetrisulfonic acid (10 g, 28.5 mmol, purity: 73% by mass), FLUOLEAD^{™} (manufactured by UBE Corporation, 44 g, 176 mmol, 6.2 equivalents), and methylene chloride (manufactured by FUJIFILM Wako Pure Chemical Corporation, 333 g) were put into a jar made of perfluoroalkoxyalkane (PFA) having a capacity of 1 L. The mixture was stirred at 40°C for 8.5 hours and then stirred at room temperature overnight.

Thereafter, potassium fluoride (manufactured by FUJIFILM Wako Pure Chemical Corporation, 22.7 g, 390 mmol, 13.7 equivalents) was added to the reaction solution in the jar, and the mixture was stirred at room temperature for 5.5 hours.

The reaction solution after the completion of stirring was filtered through a pressure filter (washed with methylene chloride, 25 g × 2 times).

In this manner, 39.4 g of a filter residue (K-FSM quantitative value: 6.4 g, 21.3 mmol, quantitative yield: 75%) and 352 g of a filtrate were obtained.

For reference, a scheme diagram of the above-described reaction is shown below.

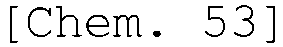

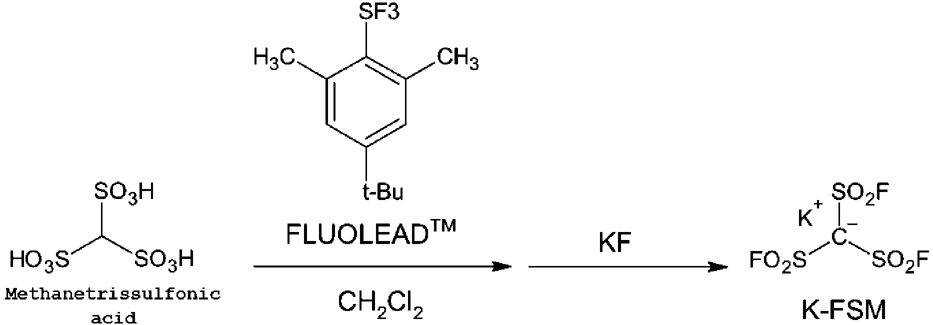

70.6 g of a filter residue (K-FSM quantitative value: 13.0 g, 41.3 mmol) combined from filter residues obtained from another batch by the same procedure and ethyl methyl carbonate (160 g) were put into a 0.5 L three-necked eggplant flask, and the mixture was stirred at room temperature for 3 hours.

The reaction solution after the completion of stirring was filtered through a pressure filter (manufactured by KISHIDA CHEMICAL Co., Ltd., washed with ethyl methyl carbonate 40 g). As a result, 77.6 g of a filter residue and 185 g of a filtrate were obtained. Then, the filtrate was subjected to distillation under reduced pressure (degree of vacuum < 2 kPa, oil bath: 55°C) to remove the solvent and dry the residue.

In this manner, K-FSM (11.6 g, quantitative value: 36.2 mmol, internal standard substance: trifluoromethylbenzene, purity: 94% by mass, process yield: 88%, overall yield: 66%) in which the amount of KF as an impurity was reduced was obtained.

The ¹⁹F-NMR analysis results of the obtained K-FSM are shown below.

¹⁹F-NMR (CD₃CN, standard substance: trifluoromethylbenzene): 70.2 ppm (s, 3F)

### [Synthesis Example 3: Synthesis of PAG-1] (Chemical structure of PAG-1 is shown below)

K-FSM (3.03 g, 10 mmol, purity: 94% by mass), triphenylsulfonium chloride (TPSCL, manufactured by Tokyo Chemical Industry Co., Ltd., 2.98 g, 10 mmol, 1.06 equivalents), chloroform (manufactured by FUJIFILM Wako Pure Chemical Corporation, 11.15 g), and ion-exchanged water (12.18 g) were put into a 200 mL three-necked eggplant flask, and the mixture was stirred at room temperature for 4 hours. Thereafter, chloroform (10.36 g) and ion-exchanged water (12.98 g) were added thereto, and the mixture was further stirred for 5 minutes.

The reaction solution after the completion of stirring was transferred to a 200 mL separatory funnel and allowed to stand for 10 minutes. Thereafter, the mixture was separated into two layers to obtain 31.91 g of an aqueous layer and 22.08 g of an organic layer.

Subsequently, ion-exchanged water (27.50 g) was added to the obtained organic layer, the mixture was stirred for 10 minutes, and allowed to stand for 10 minutes in a 200 mL separatory funnel. Thereafter, the mixture was separated into two layers to obtain 27.49 g of an aqueous layer and 20.68 g of an organic layer. Further, ion-exchanged water (26.47 g) was added to the obtained organic layer, the mixture was stirred for 10 minutes, and allowed to stand for 10 minutes in a 200 mL separatory funnel. Thereafter, the mixture was separated into two layers to obtain 27.03 g of an aqueous layer and 19.46 g of an organic layer. Then, the organic layer was subjected to distillation under reduced pressure (degree of vacuum < 2 kPa, oil bath: 50°C) to remove the solvent and dry the organic layer.

In this manner, PAG-1 (5.46 g, quantitative value: 9.38 mmol, purity: 99% by mass, yield: 93%) was obtained.

The results of nuclear magnetic resonance analysis of the obtained PAG-1 are shown below.

¹H-NMR(CDCl₃, standard substance: TMS): 7.63 to 7.82 ppm (m, 15H)

¹⁹F-NMR (CDCl₃, standard substance: CF₃CH₂OH): 71.7 ppm (s, 3F)

For reference, a scheme diagram of the above-described reaction is shown below.

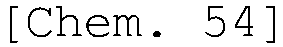

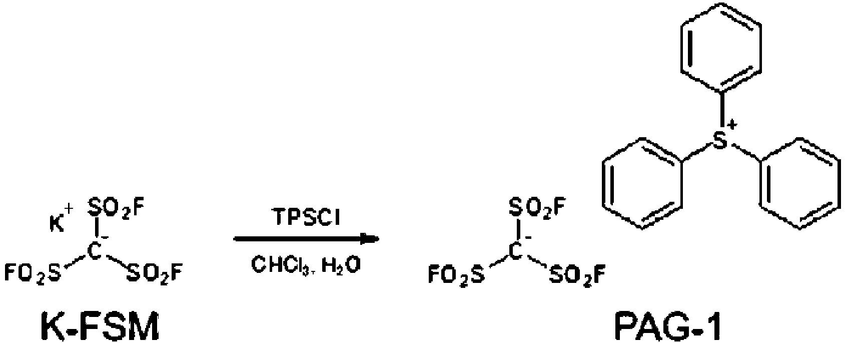

### [Synthesis Example 4: Synthesis of PAG-2]

K-FSM (1.06 g, 3.3 mmol, purity: 94% by mass), 1-adamantane methanol (manufactured by Tokyo Chemical Industry Co., Ltd., 0.55 g, 3.3 mmol, 1.0 equivalent), sodium hydride (manufactured by FUJIFILM Wako Pure Chemical Corporation, 60 wt% (mineral oil product), 0.27 g, 6.6 mmol, 2.0 equivalents), and acetonitrile (manufactured by FUJIFILM Wako Pure Chemical Corporation, 50.28 g) were placed in a 200 mL three-neck flask, and the mixture was stirred at room temperature for 20 hours.

The reaction solution after completion of the stirring was filtered through a syringe filter (pore size: 0.22 µm, made of polytetrafluoroethylene (PTFE)) to obtain 52 g of a filtrate. The filtrate was added dropwise to a 1 M hydrochloric acid aqueous solution (30.33 g), and the mixture was stirred at room temperature for 30 minutes. Thereafter, heptane (66.60 g) was added to the filtrate, and the mixture was stirred for 15 minutes. Thereafter, the reaction solution was transferred to a 500 mL separatory funnel and allowed to stand for 5 minutes. By this standing, the reaction solution was separated into three layers, and 67.01 g of a heptane layer, 43.47 g of an acetonitrile layer, and 35.26 g of an aqueous layer were obtained.

Triphenylsulfonium chloride (TPSCl, manufactured by Tokyo Chemical Industry Co., Ltd., 1.51 g, 5.0 mmol, 1.5 equivalents), chloroform (152.76 g), and ion-exchanged water (100.01 g) were added to the obtained acetonitrile layer and aqueous layer, and the mixture was stirred for 1 hour. The reaction solution after completion of the stirring was transferred to a 500 mL separatory funnel and allowed to stand for 5 minutes. By this standing, the reaction solution was separated into two layers, and 141.52 g of an aqueous layer and 188.47 g of an organic layer were obtained.

Subsequently, ion-exchanged water (101.14 g) was added to the obtained organic layer, the mixture was stirred for 10 minutes, and allowed to stand for 5 minutes in a 500 mL separatory funnel. By this standing, the mixture was separated into two layers, and 110.81 g of an aqueous layer and 175.89 g of an organic layer were obtained.

Subsequently, ion-exchanged water (99.59 g) was added to the obtained organic layer, the mixture was stirred for 10 minutes, and allowed to stand for 5 minutes in a 500 mL separatory funnel. By this standing, the mixture was separated into two layers, and 106.15 g of an aqueous layer and 166.85 g of an organic layer were obtained. Then, the organic layer was subjected to distillation under reduced pressure (degree of vacuum < 2 kPa, oil bath: 50°C) to remove the solvent and dry the organic layer.

In this manner, PAG-2 (1.92 g, quantitative value: 2.84 mmol, purity: 97% by mass, yield: 85%) was obtained.

The results of the nuclear magnetic resonance analysis of the obtained PAG-2 are shown below.

¹H-NMR (CDCl₃, standard substance: TMS): 1.58 to 1.96 ppm (m, 15H), 3.78 ppm (s, 2H), 7.63 to 7.82 ppm (m, 15H)

¹⁹F-NMR (CDCl₃, standard substance: CF₃CH₂OH): 72.1 ppm (s, 2F)

For reference, a scheme diagram of the above-described reaction is shown below. In the following, Ad represents an adamantyl group.

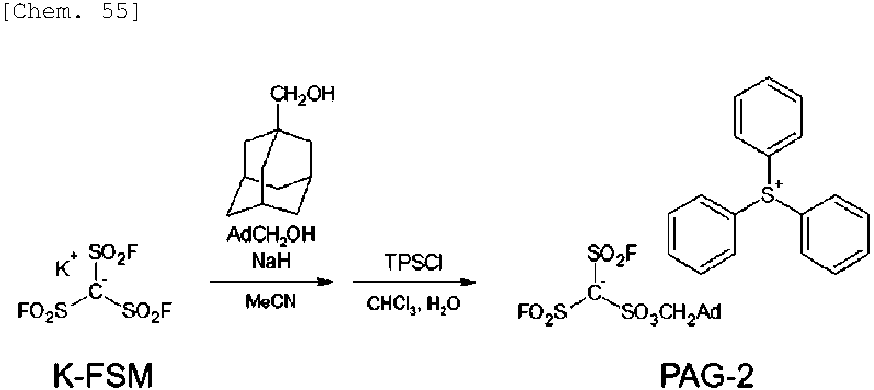

### [Synthesis Example 5: Synthesis of PAG-3 (for comparison)]

Potassium tris(trifluoromethanesulfonyl)methanide (manufactured by Tokyo Chemical Industry Co., Ltd., 4.6 g, 10 mmol, purity: 98 wt%), triphenylsulfonium chloride (TPSCl, manufactured by Tokyo Chemical Industry Co., Ltd., 2.98 g, 10 mmol, 1.06 equivalents), chloroform (manufactured by FUJIFILM Wako Pure Chemical Corporation, 11.15 g), and ion-exchanged water (12.18 g) were charged into a 200 mL three-necked eggplant flask, and the mixture was stirred at room temperature for 4 hours. Thereafter, chloroform (10.36 g) and ion-exchanged water (12.98 g) were added thereto, and the mixture was further stirred for 5 minutes.

The reaction solution after the completion of stirring was transferred to a 200 mL separatory funnel and allowed to stand for 10 minutes. Thereafter, the mixture was separated into two layers to obtain 31.91 g of an aqueous layer and 22.08 g of an organic layer. Subsequently, ion-exchanged water (27.50 g) was added to the obtained organic layer, the mixture was stirred for 10 minutes, and allowed to stand for 10 minutes in a 200 mL separatory funnel. Thereafter, the mixture was separated into two layers to obtain 27.49 g of an aqueous layer and 20.68 g of an organic layer. Further, ion-exchanged water (26.47 g) was added to the obtained organic layer, the mixture was stirred for 10 minutes, and allowed to stand for 10 minutes in a 200 mL separatory funnel. Thereafter, the mixture was separated into two layers to obtain 27.03 g of an aqueous layer and 19.46 g of an organic layer. Then, the organic layer was subjected to distillation under reduced pressure (degree of vacuum < 2 kPa, oil bath: 50°C) to remove the solvent and dry the organic layer.

In this manner, PAG-3 (6.11 g, quantitative value: 9.0 mmol, purity: 99% by mass, yield: 90%) was obtained.

The results of the nuclear magnetic resonance analysis of the obtained PAG-3 are shown below.

¹H-NMR (DMSO, standard substance: TMS): 7.67 to 7.85 ppm (m, 15H)

¹⁹F-NMR (DMSO, standard substance: CF₃-Ph): -77.2 ppm (s, 9F)

For reference, a scheme diagram of the above-described reaction is shown below.

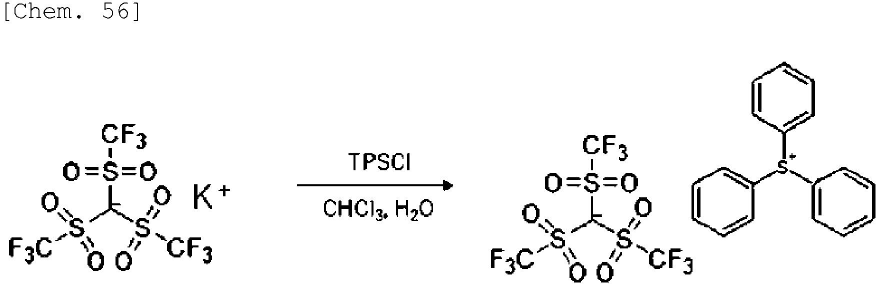

### [Preparation of comparative acid generator PAG-4]

As a comparative acid generator, an acid generator PAG-4 having the following chemical structure, manufactured by Central Glass Co., Ltd., was prepared. In the following chemical formula, Ad represents an adamantyl group.

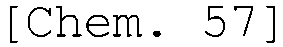

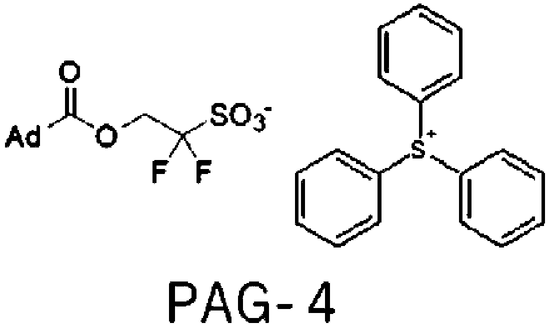

### [Synthesis Example 6: Synthesis of TBPDPS-Cl]

Magnesium (cut into flakes) (2.64 g, 0.1 mol, manufactured by Kanto Chemical Co., Inc.) was put into a 200 mL three-neck flask. A stirrer chip was put into the flask, and the mixture was stirred overnight in a nitrogen atmosphere. As a result, the oxide film on the surface of magnesium was scraped and activated. Thereafter, dehydrated tetrahydrofuran (THF, 80 ml, manufactured by Kanto Chemical Co., Inc.) was added to the flask, and the temperature was raised to 40°C. Thereafter, 1-bromo-4-tert-butylbenzene (21.3 g, 0.1 mol, manufactured by Tokyo Chemical Industry Co., Ltd.) was intermittently added thereto over 3 hours, and then the mixture was stirred at 50°C for 3 hours, the complete disappearance of 1-bromo-4-tert-butylbenzene was confirmed by gas chromatography, and then the mixture was cooled to room temperature. In this manner, a THF solution of 4-tert-butylbenzene magnesium bromide was obtained.

On the other hand, diphenyl sulfoxide (20.3 g, 0.1 mol, manufactured by Tokyo Chemical Industry Co., Ltd.), dehydrated THF (100 mL, manufactured by Kanto Chemical Co., Inc.), and chlorotrimethylsilane (14.1 g, 0.13 mol, manufactured by Tokyo Chemical Industry Co., Ltd.) were added to a 500 mL three-neck flask, and the mixture was cooled to 0°C. The THF solution of 4-tert-butylbenzene magnesium bromide synthesized above was added dropwise thereto over 1 hour while maintaining the internal temperature of the entire amount at 20°C or lower. Thereafter, the disappearance of the peak of diphenyl sulfoxide was confirmed by HPLC while stirring at 20°C. Thereafter, 15% by mass of hydrochloric acid (200 mL) was further added thereto to terminate (inactivate) the reaction.

300 mL of heptane was added to the solution after the reaction was terminated, and the mixture was stirred to separate into two layers. Thereafter, the lower layer (aqueous layer) was collected, 100 mL of IPE (isopropyl ether) was added thereto, the mixture was stirred and separated into two layers, and the aqueous layer was recovered. In this manner, an aqueous solution containing 4-t-butylphenyldiphenylsulfonium chloride (TBPDPS-Cl) was obtained with a solid content of 30.1 g and a yield of 85%.

### [Synthesis Example 7: Synthesis of MOPDPS-Cl]

The same operation as in Synthesis Example 6 was performed, except that 1-bromo-4-tert-butylbenzene was changed to 4-bromoanisole (product of Tokyo Chemical Industry Co., Ltd.). In this manner, an aqueous solution containing 4-methoxyphenyldiphenylsulfonium chloride (MOPDPS-Cl) was obtained.

### [Synthesis Example 8: Synthesis of FPDPS-Cl]

The same operation as in Synthesis Example 6 was performed, except that 1-bromo-4-tert-butylbenzene was changed to 4-bromofluorobenzene (product of Tokyo Chemical Industry Co., Ltd.). In this manner, an aqueous solution containing 4-fluorophenyldiphenylsulfonium chloride (FPDPS-Cl) was obtained.

### [Synthesis Example 9: Synthesis of Bis-sulfonium Chloride (1)]

The same operation as in Synthesis Example 6 was performed, except that 1-bromo-4-tert-butylbenzene was changed to bis(4-bromophenyl)sulfide (product of Tokyo Chemical Industry Co., Ltd.). In this manner, an aqueous solution containing (thiodi-4,1-phenylene)bis(diphenylsulfonium) dichloride was obtained.

The structures of the compounds obtained in Synthesis Examples 6 to 9 are shown below.

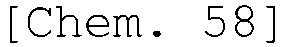

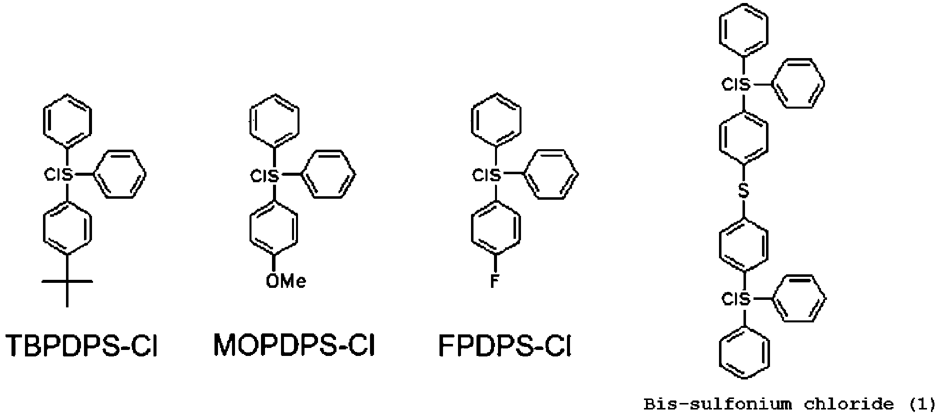

### [Synthesis Example 10: Synthesis of PAG-5]

PAG-5 was obtained by the same operation as in Synthesis Example 4, except that 1-adamantane methanol was changed to 2-adamantanone (product of Tokyo Chemical Industry Co., Ltd.).

### [Synthesis Example 11: Synthesis of PAG-6]

PAG-6 was obtained by the same operation as in Synthesis Example 4, except that 1-adamantane methanol was changed to 5-hydroxy-2-adamantanone (product of Tokyo Chemical Industry Co., Ltd.) .

### [Synthesis Example 12: Synthesis of PAG-7]

PAG-7 was obtained by the same operation as in Synthesis Example 4, except that 1-adamantane methanol was changed to 5-hydroxynorbornane-2,6-lactone (product of Tokyo Chemical Industry Co., Ltd.).

### [Synthesis Example 13: Synthesis of PAG-8]

PAG-8 was obtained by the same operation as in Synthesis Example 4, except that 1-adamantane methanol was changed to exo-norbornanol (product of Tokyo Chemical Industry Co., Ltd.).

### [Synthesis Example 14: Synthesis of PAG-9]

PAG-9 was obtained by the same operation as in Synthesis Example 4, except that triphenylsulfonium chloride (TPSCl) was changed to diphenyliodonium chloride (DPICl) (product of Tokyo Chemical Industry Co., Ltd.).

### [Synthesis Example 15: Synthesis of PAG-10]

PAG-10 was obtained by the same operation as in Synthesis Example 4, except that triphenylsulfonium chloride (TPSCl) was changed to bis(4-tert-butylphenyl)iodonium chloride (BTPICl) (product of Tokyo Chemical Industry Co., Ltd.).

### [Synthesis Example 16: Synthesis of PAG-11]

PAG-11 was obtained by the same operation as in Synthesis Example 4, except that triphenylsulfonium chloride (TPSCl) was changed to 4-t-butylphenyldiphenylsulfonium chloride synthesized in Synthesis Example 6.

### [Synthesis Example 17: Synthesis of PAG-12]

PAG-12 was obtained by the same operation as in Synthesis Example 4, except that triphenylsulfonium chloride (TPSCl) was changed to 4-methoxyphenyldiphenylsulfonium chloride synthesized in Synthesis Example 7.

### [Synthesis Example 18: Synthesis of PAG-13]

PAG-13 was obtained by the same operation as in Synthesis Example 4, except that triphenylsulfonium chloride (TPSCl) was changed to 4-fluorophenyldiphenylsulfonium chloride synthesized in Synthesis Example 8.

### [Synthesis Example 19: Synthesis of PAG-14]

PAG-14 was obtained by the same operation as in Synthesis Example 4, except that triphenylsulfonium chloride (TPSCl) was changed to (thiodi-4,1-phenylene)bis(diphenylsulfonium) dichloride synthesized in Synthesis Example 9.

The chemical structures of PAG-5 to PAG-14 are shown below. In the following, Ad is an abbreviation for an adamantyl group.

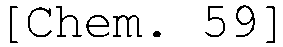

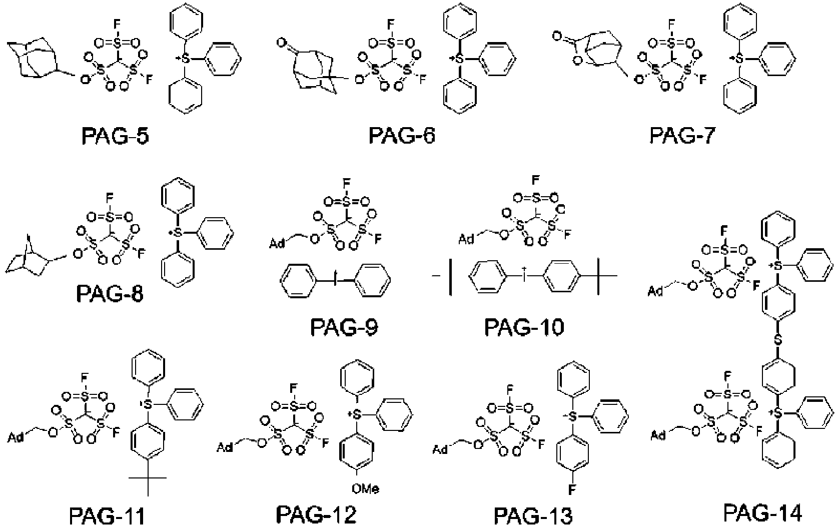

### <Evaluation: Storage stability>

0.5 g of each of PAG-1 to PAG-14 was dissolved in deuterated chloroform (4.5 g) to prepare a solution in which the concentration of the acid generator was 10% by mass.

¹⁹F-NMR was measured immediately after the preparation and after storing at room temperature for 24 hours. The NMR chart immediately after the preparation and the NMR chart after storing at room temperature for 24 hours were compared and evaluated as follows.
· In a case where a new peak was confirmed in the NMR chart after storing at room temperature for 24 hours: defective
· In a case where a new peak was not confirmed in the NMR chart after storing at room temperature for 24 hours: good

### <Evaluation: Alkali hydrolyzability>

0.5 g of each of PAG-1 to PAG-14 was mixed with 2.38% by mass of a tetramethylammonium aqueous solution (0.1 g) and deuterated chloroform (4.5 g) to prepare a solution in which the concentration of the acid generator was 10% by mass. The obtained solution was stirred at room temperature for 24 hours.

¹⁹F-NMR was measured for the solution after the completion of the stirring. The evaluation was performed as follows by comparing the NMR chart immediately after the preparation measured in <Evaluation: Storage stability> described above.
· In a case where the peak attributed to fluorine in the acid generator completely disappeared and a new peak not attributed to fluorine in the acid generator was confirmed: good
· In a case where both the peak attributed to fluorine in the acid generator and the new peak not attributed to fluorine in the acid generator could be confirmed: acceptable
· In a case where the new peak not attributed to fluorine in the acid generator was not confirmed: defective

The evaluation results are summarized in the table below.

### [Table 1]

**Table 1**

| | Acid generator | Storage stability | Alkaline hydrolyzability |
|---|---|---|---|
| Example 1-1 | PAG-1 | Good | Good |
| Example 1-2 | PAG-2 | Good | Good |
| Example 1-3 | PAG-5 | Good | Good |
| Example 1-4 | PAG-6 | Good | Good |
| Example 1-5 | PAG-7 | Good | Good |
| Example 1-6 | PAG-8 | Good | Good |
| Example 1-7 | PAG-9 | Good | Good |
| Example 1-8 | PAG-10 | Good | Good |
| Example 1-9 | PAG-11 | Good | Good |
| Example 1-10 | PAG-12 | Good | Good |
| Example 1-11 | PAG-13 | Good | Good |
| Example 1-12 | PAG-14 | Good | Good |
| Comparative Example 1-1 | PAG-3 | Good | Defective |
| Comparative Example 1-2 | PAG-4 | Good | Acceptable |

From the above-described results, it was shown that the acid generator represented by General Formula (AG1) is stable in an organic solvent, but is easily decomposed by the action of an alkali. This means that, in a case where a radiation-sensitive resin composition containing the acid generator represented by General Formula (AG1) is used to form a pattern on a substrate by exposure and an alkali development treatment, a residue derived from the acid generator is less likely to remain on the substrate, and thus the residue amount of fluorine atoms on the substrate can be reduced.

### <Preparation of radiation-sensitive resin composition (for ArF exposure)>

The above-described PAG-1 (0.8 g), the following resin A (10 g), triethylamine (0.1 g) as a quencher, propylene glycol monomethyl ether acetate (50 g) as a solvent, and γ-butyrolactone (50 g) were sufficiently mixed to prepare a solution. The following resin A is a random copolymer. This solution was filtered through a Teflon (registered trademark) filter having a pore size of 0.2 µm. In this manner, a radiation-sensitive resin composition (Example 2-1) was prepared.

In addition, radiation-sensitive resin compositions (Examples 2-2, Comparative Examples 2-1 and 2-2, and Examples 2-3 to 2-12) were prepared using PAG-2 to PAG-14 instead of PAG-1. The correspondence relationship between the example/comparative example number and the acid generator used is also referred to in the table below.

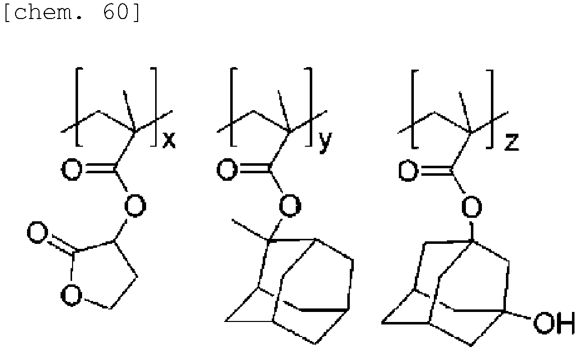

In the resin A, x = 0.4, y = 0.4, and z = 0.2.

In addition, the weight-average molecular weight Mw of the resin A is 10,600, and the dispersity Mw/Mn is 1.75.

### <Evaluation of radiation-sensitive resin composition (ArF exposure)>

An antireflective film ARC29A manufactured by Nissan Chemical Corporation was applied onto a silicon wafer having a diameter of 4 inches with a spin coater, and baked at 200°C for 60 seconds to form an antireflective film having a film thickness of 84 nm.

The radiation-sensitive resin composition was applied onto the antireflective film with a spin coater and baked at 120°C for 60 seconds to form a resin film having a film thickness of 280 nm.

The formed resin film was irradiated with light having a wavelength of 193 nm using an exposure device: ArFES-3500LDLS (manufactured by Litho Tech Japan Corporation). In this case, a photomask was not used (blank exposure). After the irradiation, the silicon wafer provided with the resin film was baked at 120°C for 60 seconds. Then, the silicon wafer was developed at 23°C for 60 seconds using a 2.38% by mass tetramethylammonium hydroxide aqueous solution. The silicon wafer after the development was rinsed with pure water.

The film thickness of the resin film remaining after the rinsing was measured with an ellipsometer M2000 (manufactured by J. A. Woollam Japan, Ltd.). The sensitivity was evaluated by setting the exposure dose at which the film thickness reduction amount was 90% or more as the minimum required exposure dose (Eₜₕ).

### <Evaluation of radiation-sensitive resin composition (evaluation of fluorine remaining on substrate)>

A radiation-sensitive resin composition was applied onto a silicon wafer having a diameter of 4 inches with a spin coater and baked at 120°C for 60 seconds. In this manner, a resin film having a film thickness of 280 nm was formed.

The formed resin film was irradiated with light having a wavelength of 193 nm using an exposure device: ArFES-3500LDLS (manufactured by Litho Tech Japan Corporation). In this case, a photomask was not used (blank exposure). In addition, the exposure dose was set to 10 mJ/cm² (irradiation amount sufficiently larger than Eₜₕ). Thereafter, the silicon wafer provided with the resin film was baked under the conditions of 120°C for 60 seconds. Then, the silicon wafer was developed at 23°C for 60 seconds using a 2.38% by mass tetramethylammonium hydroxide aqueous solution. The silicon wafer after the development was rinsed with pure water.

The silicon wafer after the rinsing was subjected to X-ray photoelectron spectroscopy (XPS) to measure an F1s spectrum on the wafer surface, and the F component intensity (unit: cps) was compared. The XPS measurement was performed using JPS-9000MA manufactured by JEOL Ltd. The measurement was performed at 10 locations by changing the location, and the degree of residue fluorine was evaluated by the average value of the intensities at the 10 locations. It can be said that the smaller the value obtained here, the smaller the residue amount of the fluorine atom-containing component on the wafer.

The results of the evaluation (ArF exposure) using the radiation-sensitive resin composition are summarized in the table below.

### [Table 2]

**Table 2**

| | Acid generator | Sensitivity evaluation (mJ/cm²) | F component intensity (cps) |
|---|---|---|---|
| Example 2-1 | PAG-1 | 2.75 | < 10 |
| Example 2-2 | PAG-2 | 4.70 | < 10 |
| Example 2-3 | PAG-5 | 4.68 | < 10 |
| Example 2-4 | PAG-6 | 4.72 | < 10 |
| Example 2-5 | PAG-7 | 4.65 | < 10 |
| Example 2-6 | PAG-8 | 4.62 | < 10 |
| Example 2-7 | PAG-9 | 4.40 | < 10 |
| Example 2-8 | PAG-10 | 4.55 | < 10 |
| Example 2-9 | PAG-11 | 4.72 | < 10 |
| Example 2-10 | PAG-12 | 4.73 | < 10 |
| Example 2-11 | PAG-13 | 4.45 | < 10 |
| Example 2-12 | PAG-14 | 4.50 | < 10 |
| Comparative Example 2-1 | PAG-3 | 2.80 | 500 |
| Comparative Example 2-2 | PAG-4 | 4.72 | 380 |

### <Preparation of radiation-sensitive resin composition (for KrF exposure)>

The above-described PAG-1 (0.8 g), the following resin B (10 g), triethylamine (0.1 g) as a quencher, propylene glycol monomethyl ether acetate (50 g) as a solvent, and γ-butyrolactone (50 g) were sufficiently mixed to obtain a solution. The resin B is a random copolymer. This solution was filtered through a Teflon (registered trademark) filter having a pore size of 0.2 µm. In this manner, a radiation-sensitive resin composition (Example 3-1) was prepared.

In addition, radiation-sensitive resin compositions (Examples 3-2 and Comparative Example 3-1) were prepared using PAG-2 and PAG-4 instead of PAG-1.

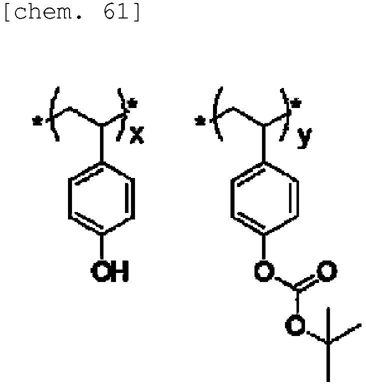

In the resin B, x = 0.3 and y = 0.7.

In addition, the weight-average molecular weight Mw of the resin B was 11,600, and the dispersity Mw/Mn was 1.95.

### <Preparation of radiation-sensitive resin composition (for KrF exposure)>

The above-described PAG-1 (0.8 g), a calixarene resin (10 g), triethylamine (0.1 g) as a quencher, propylene glycol monomethyl ether acetate (50 g) as a solvent, and γ-butyrolactone (50 g) were sufficiently mixed to obtain a solution. This solution was filtered through a Teflon (registered trademark) filter having a pore size of 0.2 µm. In this manner, a radiation-sensitive resin composition (Example 4-1) was prepared. Here, the calixarene resin was prepared with reference to J. Photopolym. Sci. Technol., Vol. 25, 5, 2012, pp. 587 to 592. The weight-average molecular weight Mw was 3,300, the dispersity Mw/Mn was 1.1, and the protection rate of the phenolic hydroxy group was 60%.

In addition, radiation-sensitive resin compositions (Examples 4-2 and Comparative Example 4-1) were prepared using PAG-2 and PAG-4 instead of PAG-1.

### <Evaluation of radiation-sensitive resin composition (KrF exposure)>

The radiation-sensitive resin composition (for KrF exposure) was applied onto a silicon wafer having a diameter of 4 inches with a spin coater, and baked at 100°C for 60 seconds to form a resin film having a film thickness of 280 nm.

Exposure device: The formed resin film was irradiated with light having a wavelength of 248 nm using a VUVES-4700i (manufactured by Litho Tech Japan Corporation). In this case, a photomask was not used (blank exposure). After the irradiation, the silicon wafer provided with the resin film was baked at 120°C for 60 seconds. Then, the silicon wafer was developed at 23°C for 60 seconds using a 2.38% by mass tetramethylammonium hydroxide aqueous solution. The silicon wafer after the development was rinsed with pure water.

The film thickness of the resin film remaining after the rinsing was measured with an ellipsometer M2000 (manufactured by J. A. Woollam Japan, Ltd.). The sensitivity was evaluated by setting the exposure dose at which the film thickness reduction amount was 90% or more as the minimum required exposure dose (Eₜₕ).

### <Evaluation of radiation-sensitive resin composition (evaluation of fluorine remaining on substrate)>

The radiation-sensitive resin composition (for KrF exposure) was applied onto a silicon wafer having a diameter of 4 inches with a spin coater, and baked at 100°C for 60 seconds. In this manner, a resin film having a film thickness of 280 nm was formed.

Exposure device: The formed resin film was irradiated with light having a wavelength of 248 nm using a VUVES-4700i (manufactured by Litho Tech Japan Corporation). In this case, a photomask was not used (blank exposure). In addition, the exposure dose was set to 10 mJ/cm² (irradiation amount sufficiently larger than Eₜₕ). Thereafter, the silicon wafer provided with the resin film was baked under the conditions of 120°C for 60 seconds. Then, the silicon wafer was developed at 23°C for 60 seconds using a 2.38% by mass tetramethylammonium hydroxide aqueous solution. The silicon wafer after the development was rinsed with pure water.

The silicon wafer after the rinsing was subjected to X-ray photoelectron spectroscopy (XPS) to measure an F1s spectrum on the wafer surface, and the F component intensity (unit: cps) was compared. The XPS measurement was performed using JPS-9000MA manufactured by JEOL Ltd. The measurement was performed at 10 locations by changing the location, and the degree of residue fluorine was evaluated by the average value of the intensities at the 10 locations. It can be said that the smaller the value obtained here, the smaller the residue amount of the fluorine atom-containing component on the wafer.

The results of the evaluation (KrF exposure) using the radiation-sensitive resin composition are summarized in the table below.

### [Table 3]

**Table 3**

| | Acid generator | Sensitivity evaluation (mJ/cm²) | F component intensity (cps) |
|---|---|---|---|
| Example 3-1 | PAG-1 | 10.5 | < 10 |
| Example 3-2 | PAG-2 | 13.1 | < 10 |
| Comparative Example 3-1 | PAG-4 | 11.0 | 340 |
| Example 4-1 | PAG-1 | 6.03 | < 10 |
| Example 4-2 | PAG-2 | 7.37 | < 10 |
| Comparative Example 4-1 | PAG-4 | 6.25 | 330 |

From the results shown in Tables 2 and 3, the following can be said.
(i) The sensitivity of the radiation-sensitive resin composition containing the acid generators (PAG-1, PAG-2, and PAG-5 to PAG-14) corresponding to General Formula (AG1) was the same as the sensitivity of the radiation-sensitive resin composition containing the acid generators (PAG-3 and PAG-4) in the related art. This is considered to be because the acid strength of the generated acid is sufficiently strong due to the fact that PAG-1, PAG-2, and PAG-5 to PAG-14 have an electron-withdrawing fluorosulfonyl structure.
(ii) In a case where the lithography process including the development process using an alkaline aqueous solution was performed using the radiation-sensitive resin composition containing the acid generator corresponding to General Formula (AG1), the residue amount of the fluorine atom-containing component on the substrate could be suppressed. This is considered to be due to the fact that the acid generated from PAG-1, PAG-2, and PAG-5 to PAG-14 was hydrolyzed by the alkaline component.
(iii) In the radiation-sensitive resin compositions of Examples 2-1 to 12, 3-1, 3-2, 4-1, and 4-2 containing PAG-1, PAG-2, and PAG-5 to PAG-14, the residue amount of the fluorine atom on the substrate after the alkali development treatment was suppressed even though the acid generator contained a fluorine atom for achieving higher sensitivity. This is a departure from the trade-off in the related art.
(iv) As shown in Table 3, even in the KrF exposure (wavelength: 248 nm), the sensitivity of the radiation-sensitive resin composition containing the acid generators (PAG-1 and PAG-2) corresponding to General Formula (AG1) was the same as the sensitivity of the radiation-sensitive resin composition containing the acid generator (PAG-4) in the related art. From this result, the compound (acid generator) represented by General Formula (AG1) can also be used for the KrF resist. In addition, since the KrF sensitivity and the EUV sensitivity are different (Journal of Photopolymer Science and Technology Volume 37, Number 1 (2024) 89-93), the present invention can also be expected for EUV resist applications.

Incidentally, in the present specification, Examples of the radiation-sensitive resin composition according to the second embodiment are not described. However, in consideration of the above-described mechanism that the residue amount of the fluorine-containing component can be suppressed by the decomposition of the acid generated from the acid generator by the alkaline developer, the results supporting this mechanism (see <Evaluation: Alkaline hydrolyzability> described above), and the like, it is considered that the residue amount of the fluorine-containing component can also be suppressed in the radiation-sensitive resin composition according to the second embodiment.

This application claims priority based on Japanese Patent Application No. 2023-202572 filed on November 30, 2023, the entirety of the disclosure of which is incorporated herein.

## Claims

1. A radiation-sensitive resin composition comprising:
an acid generator represented by General Formula (AG1), wherein 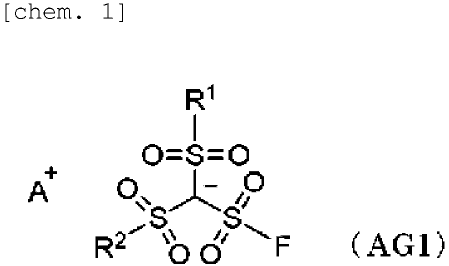
in General Formula (AG1),
R¹ and R² each independently represent a fluorine atom or - OR, where R is a monovalent organic group, and
A⁺ is a counter cation.

2. The radiation-sensitive resin composition according to claim 1, further comprising:
a resin which is decomposed by an action of an acid to change solubility in a developer.

3. The radiation-sensitive resin composition according to claim 1, further comprising:
a component that increases in molecular weight due to an action of an acid and changes solubility in a developer.

4. The radiation-sensitive resin composition according to any one of claims 1 to 3,
wherein R¹ and R² are both fluorine atoms.

5. The radiation-sensitive resin composition according to any one of claims 1 to 3,
wherein one of R¹ or R² is a fluorine atom and the other is -OR.

6. The radiation-sensitive resin composition according to any one of claims 1 to 3,
wherein at least one of R¹ or R² is -OR, and R includes a cyclic skeleton.

7. The radiation-sensitive resin composition according to any one of claims 1 to 3,
wherein A⁺ is at least any cation selected from the group consisting of a sulfonium cation and an iodonium cation.

8. A pattern formation method comprising:
forming a film using the radiation-sensitive resin composition according to any one of claims 1 to 3;
irradiating the film with radiation; and
developing the film irradiated with the radiation.

9. A method for manufacturing an electronic device, the method comprising:
the pattern formation method according to claim 8.

10. An acid generator represented by General Formula (AG1), wherein 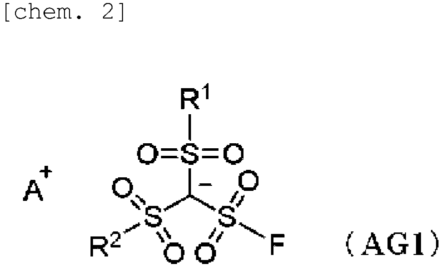 in General Formula (AG1),
R¹ and R² each independently represent a fluorine atom or - OR, where R is a monovalent organic group, and
A⁺ is a counter cation.

11. The acid generator according to claim 10,
wherein both R¹ and R² represent a fluorine atom.

12. The acid generator according to claim 10 or 11,
wherein one of R¹ or R² represents a fluorine atom and the other represents -OR.

13. The acid generator according to claim 10,
wherein at least one of R¹ or R² represents -OR, and R includes a cyclic skeleton.

14. The acid generator according to claim 10 or 11,
wherein A⁺ represents at least any cation selected from the group consisting of a sulfonium cation and an iodonium cation.

15. A method for producing a compound, the method comprising:
reacting a compound represented by General Formula (ag1) with a compound represented by General Formula A₁⁺X⁻ (A₁⁺ is a sulfonium cation or an iodonium cation, and X⁻ is a halide ion, a sulfonate ion, a sulfate ion, a phosphate ion, a hydroxide ion, or a carboxylate ion) to produce a compound represented by General Formula (AG1-1), wherein 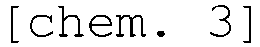 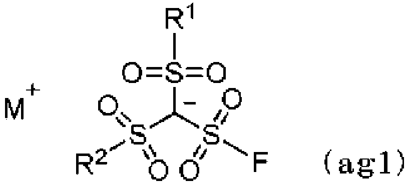
in General Formula (ag1),
M⁺ is a metal cation,
R¹ and R² each independently represent a fluorine atom or - OR, where R is a monovalent organic group, 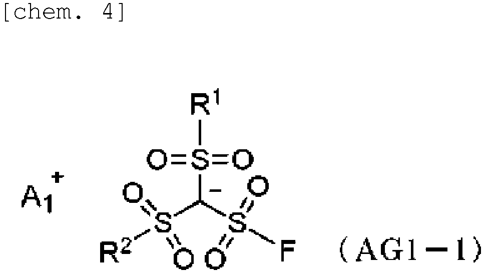
in General Formula (AG1-1),
a definition of A₁⁺ is as described above,
R¹ and R² each independently represent a fluorine atom or - OR, where R is a monovalent organic group.

16. A method for producing a compound, the method comprising:
reacting a compound represented by General Formula (M-FSM) with a compound represented by R-OH, where R is a monovalent organic group, to obtain a compound represented by General Formula (ag1), wherein 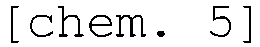 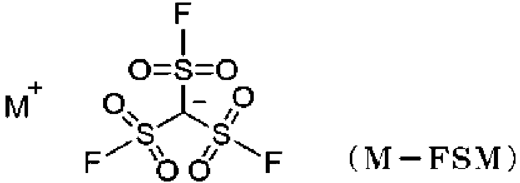
in General Formula (M-FSM),
M⁺ is a metal cation, 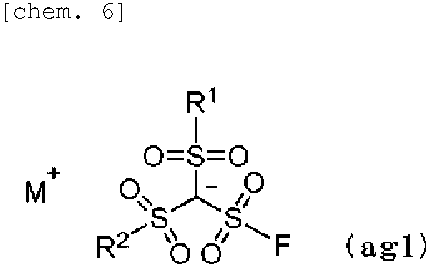
in General Formula (ag1),
M⁺ is a metal cation,
R¹ and R² each independently represent a fluorine atom or - OR, where at least one of R¹ or R² is -OR, and R is a monovalent organic group.

17. A compound represented by General Formula (AG1), wherein 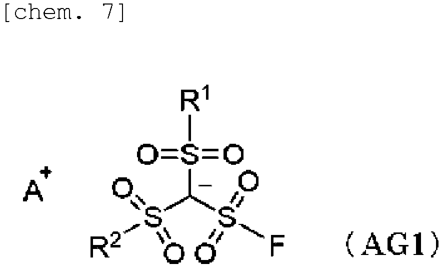 in General Formula (AG1),
R¹ and R² each independently represent a fluorine atom or - OR, where R is a monovalent organic group, and
A⁺ is a counter cation.

18. The compound according to claim 17,
wherein both R¹ and R² represent a fluorine atom.

19. The compound according to claim 17 or 18,
wherein one of R¹ or R² represents a fluorine atom, and the other represents -OR.

20. The compound according to claim 17,
wherein at least one of R¹ or R² represents -OR, and R includes a cyclic skeleton.

21. The compound according to claim 17 or 18,
wherein A⁺ represents at least any cation selected from the group consisting of a sulfonium cation and an iodonium cation.

22. A method for generating an acid, the method comprising:
irradiating the compound according to claim 17 or 18 with radiation to generate an acid.
